(19) Europäisches Patentamt
European Patent Office
Office européen des brevets

(11) Veröffentlichungsnummer: **0 085 892**
**B1**

# (12) EUROPÄISCHE PATENTSCHRIFT

(45) Veröffentlichungstag der Patentschrift: **18.12.85**

(21) Anmeldenummer: **83100677.0**

(22) Anmeldetag: **26.01.83**

(51) Int. Cl.⁴: **C 07 D 401/06**, C 07 D 451/02, A 61 K 31/55 // C07D243/38, C07F9/40

(54) **Substituierte Dibenzodiazepinone, Verfahren zu ihrer Herstellung und sie enthaltende Arzneimittel.**

(30) Priorität: **06.02.82 DE 3204157**

(43) Veröffentlichungstag der Anmeldung: **17.08.83 Patentblatt 83/33**

(45) Bekanntmachung des Hinweises auf die Patenterteilung: **18.12.85 Patentblatt 85/51**

(84) Benannte Vertragsstaaten: **AT BE CH DE FR IT LI LU NL SE**

(56) Entgegenhaltungen:
**EP - A - 0 031 219**
**EP - A - 0 044 989**
**DE - A - 1 795 176**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Trummlitz, Günter, Dr., Dipl.-Chem., Buchenweg 27, D-7951 Warthausen (DE)**
Erfinder: **Engel, Wolfhard, Dr., Dipl.-Chem., Mozartstrasse 13, D-7950 Biberach 1 (DE)**
Erfinder: **Eberlein, Wolfgang, Dr., Dipl.-Chem., Obere Au 6, D-7950 Biberach 1 (DE)**
Erfinder: **Schmidt, Günther, Dr., Dipl.-Chem., Johann-Seb.-Bach-Strasse 27, D-7950 Biberach 1 (DE)**
Erfinder: **Hammer, Rudolf, Dr., Via Fabio Filzi 33, Milano (IT)**
Erfinder: **Del Soldato, Piero, Dr., Via E. Toti 22, Monza (IT)**

**EP 0 085 892 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte Dibenzodiazepinone, Verfahren zu ihrer Herstellung und sie enthaltende Arzneimittel.

In der deutschen Offenlegungsschrift DE-OS 1 795 176 werden bestimmte Dibenzodiazepinone mit einer ulkushemmenden und sekretionshemmenden Wirkung beschrieben. Aus der US-Patentschrift US-PS 3 953 430 sind substituierte Dibenzodiazepine mit antidepressiver und analgetischer Wirkung bekannt.

Es wurden nun Dibenzodiazepinone mit neuartigen Aminoacylresten gefunden, die gegenüber den oben erwähnten Verbindungen interessante und überlegene pharmakologische Wirkungen aufweisen.

Gegenstand der Erfindung sind:

a.) substituierte Dibenzodiazepinone der allgemeinen Formel I

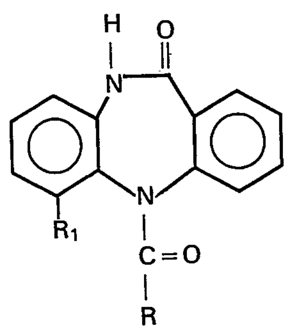

(I)

worin $R_1$ ein Wasserstoff- oder Chloratom und R einen — im heterocyclischen Sechsring gegebenenfalls durch eine oder zwei weitere Methylgruppen substituierten — (1-Methyl-4-piperidinyl)-methyl-, (1-Methyl-1,2,5,6-tetrahydro-4-pyridinyl)-methyl-, 1-Methyl-1,2,5,6-tetrahydro-4-pyridinyl-, (1-Methyl-4-piperidinyliden)-methyl-, (2,3-Dehydro-8-methyl-8-aza-bicyclo[3,2,1]oct-3-yl)-methyl-, (8-Methyl-8-aza-bicyclo[3,2,1]oct-3-yliden)-methyl- oder den endo- oder exo-(8-Methyl-8-aza-bicyclo[3,2,1]-oct-3-yl)-methyl-Rest bedeuten, sowie ihre Säureadditionssalze.

Die Verbindungen der allgemeinen Formel I können nach Umsetzung mit anorganischen oder organischen Säuren auch in Form ihrer physiologisch verträglichen Salze vorliegen. Als Säuren haben sich hierzu beispielsweise Salzsäure, Bromwasserstoffsäure, Schwefelsäure, Phosphorsäure, Weinsäure, Fumarsäure, Citronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

5,10-Dihydro-5-[(1-methyl-4-piperidinyl)acetyl]-11H-dibenzo[b,e][1,4]diazepin-11-on

5,10-Dihydro-5-[(1,3-dimethyl-4-piperidinyl)acetyl]-11H-dibenzo[b,e][1,4]diazepin-11-on

5,10-Dihydro-5-[(1,2-dimethyl-4-piperidinyl)acetyl]-11H-dibenzo[b,e][1,4]diazepin-11-on

cis-5,10-Dihydro-5-[(1,2-dimethyl-4-piperidinyl)-acetyl]-11H-dibenzo[b,e][1,4]diazepin-11-on

trans-5,10-Dihydro-5-[(1,2-dimethyl-4-piperidinyl)acetyl]-11H-dibenzo[b,e][1,4]diazepin-11-on

cis-5,10-Dihydro-5-[(1,3-dimethyl-4-piperidinyl)-acetyl]-11H-dibenzo[b,e][1,4]diazepin-11-on

trans-5,10-Dihydro-5-[(1,3-dimethyl-4-piperidinyl)acetyl]-11H-dibenzo[b,e][1,4]diazepin-11-on

5,10-Dihydro-5-[(1-methyl-1,2,5,6-tetrahydro-4-pyridinyl)acetyl]-11H-dibenzo[b,e][1,4]diazepin-11-on

5,10-Dihydro-5-[(1,2-dimethyl-1,2,5,6-tetrahydro-4-pyridinyl)acetyl]-11H-dibenzo[b,e][1,4]diazepin-11-on

5,10-Dihydro-5-[(1,3-dimethyl1,2,5,6-tetrahydro-4-pyridinyl)acetyl]-11H-dibenzo[b,e][1,4]diazepin-11-on

5,10-Dihydro-5-[(1,5-dimethyl-1,2,5,6-tetrahydro-4-pyridinyl)acetyl]-11H-dibenzo[b,e][1,4]diazepin-11-on

5,10-Dihydro-5-[(1,6-dimethyl-1,2,5,6-tetrahydro-4-pyridinyl)acetyl]-11H-dibenzo[b,e][1,4]diazepin-11-on

5,10-Dihydro-5-[(1,2,6-trimethyl-1,2,5,6-tetrahydro-4-pyridinyl)carbonyl]-11H-dibenzo[b,e][1,4]-diazepin-11-on

cis-5,10-Dihydro-5-[(1,2,6-trimethyl-1,2,5,6-tetrahydro-4-pyridinyl)carbonyl]-11H-dibenzo[b,e]-[1,4]diazepin-11-on

trans-5,10-Dihydro-5-[(1,2,6-trimethyl1,2,5,6-tetrahydro-4-pyridinyl)carbonyl]-11H-dibenzo-[b,e][1,4]diazepin-11-on

5,10-Dihydro-5-[(1-methyl-1,2,5,6-tetrahydro-4-pyridinyl)carbonyl]-11H-dibenzo[b,e][1,4]diazepin-11-on

5,10-Dihydro-5-[(1,2-dimethyl-1,2,5,6-tetrahydro-4-pyridinyl)carbonyl]-11H-dibenzo[b,e][1,4]diazepin-11-on

5,10-Dihydro-5-[(1,3-dimethyl1,2,5,6-tetrahydro-4-pyridinyl)carbonyl]-11H-dibenzo[b,e][1,4]diazepin-11-on

5,10-Dihydro-5-[(1,5-dimethyl-1,2,5,6-tetrahydro-4-pyridinyl)carbonyl]-11H-dibenzo[b,e][1,4]diazepin-11-on

5,10-Dihydro-5-[(1,6-dimethyl-1,2,5,6-tetrahydro-4-pyridinyl)carbonyl]-11H-dibenzo[b,e][1,4]diazepin-11-on

endo-5,10-Dihydro-5-[(8-methyl-8-azabicyclo-[3,2,1]oct-3-yl)-acetyl]-11H-dibenzo[b,e][1,4]diazepin-11-on

exo-5,10-Dihydro-5-[(8-methyl-8-azabicyclo-[3,2,1]oct-3-yl)acetyl]-11H-dibenzo[b,e][1,4]diazepin-11-on

5,10-Dihydro-5-[(1-methyl-4-piperidinyliden)-acetyl]-11H-dibenzo[b,e][1,4]diazepin-11-on

6-Chlor-5,10-dihydro-5-[(1-methyl-4-piperidinyl)acetyl]-11H-dibenzo[b,e][1,4]diazepin-11-on

6-Chlor-5,10-dihydro-5-[(1-methyl-1,2,5,6-tetrahydro-4-pyridinyl)acetyl]-11H-dibenzo[b,e][1,4]diazepin-11-on

6-Chlor-5,10-dihydro-5-[(1-methyl-1,2,5,6-tetrahydro-4-pyridinyl)carbonyl]-11H-dibenzo[b,e][1,4]-diazepin-11-on

endo-6-Chlor-5,10-dihydro-5-[(8-methyl-8-azabicyclo[3,2,1]oct-3-yl)acetyl]-11H-dibenzo[b,e]-[1,4]diazepin-11-on

exo-6-Chlor-5,10-dihydro-5-[(8-methyl-8-aza-

bicyclo[3,2,1]oct-3-yl)acetyl]-11H-dibenzo[b,e]-[1,4]diazepin-11-on

6-Chlor-5,10-dihydro-5-[(1,3-dimethyl-1,2,5,6--tetrahydro-4-pyridinyl)carbonyl]-11H-dibenzo-[b,e][1,4]diazepin-11-on.

b.) Arzneimittel, die ein oder mehrere Dibenzodiazepinone der allgemeinen Formel I enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragees, Kapseln oder Teezubereitungen einarbeiten. Die Tagesdosis liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die substituierten Dibenzodiazepinone der allgemeinen Formel I und ihre Säureadditionssalze besitzen wertvolle Eigenschaften, die sie gewerblich verwertbar machen, insbesondee sind sie durch eine ausgezeichnete Magen- und Darmschutzwirkung bei Warmblütern gekennzeichnet, sie hemmen z.B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der substituierten Dibenzodiazepinone der allgemeinen Formel I und ihrer pharmakologisch, d.h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise können mit ihnen akute und chronische Ulcera ventriculi und duodeni, Gastritis oder hyperacider Reizmagen bei Mensch oder Tier behandelt werden.

Sollen die erfindungsgemässen substituierten Dibenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Sekretionshemmer, wie $H_2$-Blocker, z.B. Cimetidin, Ranitidin; Magen- und Darmtherapeutika, z.B. Metoclopramid, Bromoprid, Tiaprid; Tranquilizer, wie Benzodiazepine, beispielsweise Diazepam, Oxazepam; Spasmolytika, z.B. Bietamiverin, Camylofin; Anticholinergica, z.B. Oxyphencyclimin, Phencarbamid; Glucocorticoide, wie Prednisolon, Fluorcortolon, Betamethason; nicht-steroidale Antiphlogistika, wie Arylessigsäuren und -propionsäuren, Heteroarylessigsäuren und -propionsäuren, Benzothiazincarboxamiddioxide, Pyrazolidindione, Chinazolinone, z.B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon; Lokalanaesthetika, beispielsweise Tetracain, Procain; gegebenenfalls auch Fermente, Vitamine, Aminosäuren usw. enthalten.

c.) Verfahren zur Herstellung der substituierten Dibenzodiazepinone der allgemeinen Formel I

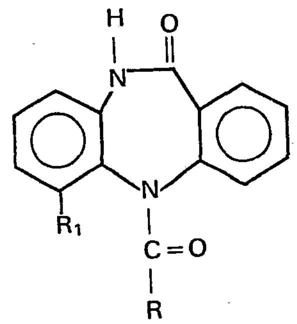

(I)

mit den eingangs erwähnten Bedeutungen für R und $R_1$, sowie ihrer Säureadditionssalze.

Die Verbindungen der allgemeinen Formel I lassen sich wie folgt darstellen:

a.) Alle Verbindungen der allgemeinen Formel I können dadurch erhalten werden, dass man Dibenzodiazepinone der allgemeinen Formel II

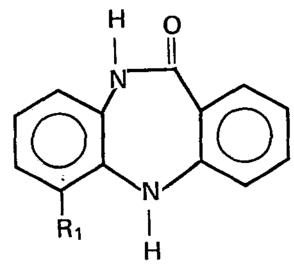

(II)

worin

$R_1$ die oben angegebene Bedeutung hat, mit Säurederivaten der allgemeinen Formel III

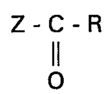

(III)

worin R die oben angegebene Bedeutung hat und Z eine nucleofuge Gruppe bzw. Abgangsgruppe darstellt, acyliert.

Die Umsetzung der Verbindungen der allgemeinen Formel II mit den Säurederivaten der allgemeinen Formel III erfolgt in an sich bekannter Weise. Die Abgangsgruppe Z ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester-, -anhydride oder gemischte -anhydride, wie sie aus Salzen der entsprechenden Säure (Z = OH) und Säurechloriden, wie Phosphoroxychlorid, Diphosphorsäuretetrachlorid oder Chlorameisensäureester, gebildet werden oder die bei der Umsetzung von III (Z = OH) mit N-Alkyl-2-halogen-pyridiniumsalzen entstehenden N-Alkyl-2-acyloxy-pyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise genannt Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-dimethylaminopyridin; oder Natriumhydrid. Die Reaktion wird bei Temperaturen zwischen —25 und 130°C in einem inerten Lösungsmittel durchgeführt. Als inerte Lösungsmittel kommen beispielsweise in Frage chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon. Die Reaktionszeiten betragen je nach Menge und Art des eingesetzten Acylierungsmittels der allgemeinen Formel III zwischen 15 Minuten und 80 Stunden. Es ist nicht nötig, die Verbindungen der allgemeinen Formel III in reiner Form herzustellen, sie können vielmehr im Reaktionsansatz in bekannter Weise in situ erzeugt werden.

b.) Solche Verbindungen der allgemeinen Formel I, worin R einen im heterocyclischen Sechsring gegebenenfalls durch eine oder zwei weitere Methylgruppen substituierten (1-Methyl-1,2,5,6-tetrahydro-4-pyridinyl)methyl- oder 1-Methyl-1,2,5,6-tetrahydro-4-pyridinyl-Rest bedeutet, werden auch dadurch erhalten, dass man ein Dibenzodiazepinon der allgemeinen Formel II mit Acylierungsmitteln der allgemeinen Formel IV

$$Z - \underset{\underset{\displaystyle O}{\|}}{C} - R_p \qquad (IV)$$

in der Z die oben für die Formel III angegebenen Bedeutungen hat und $R_p$ ein gegebenfalls methyl- oder dimethylsubstituierter 4-Pyridinyl- oder (4-Pyridinyl)methyl-Rest ist, zur Reaktion bringt.

Die Acylierung gelingt unter den bei a) ausgeführten Bedingungen, jedoch wird die Umsetzung in siedendem Dioxan in Gegenwart von Pyridin, 4-Dimethylamino-pyridin oder Triethylamin bevorzugt.

Die so erhaltenen Zwischenverbindungen der allgemeinen Formel V

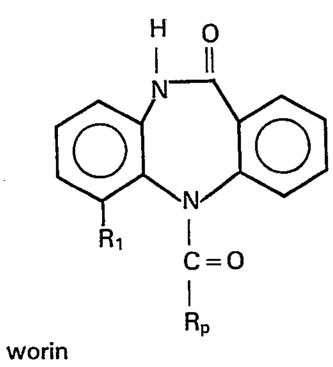

(V)

worin

$R_1$ und $R_p$ die oben angegebenen Bedeutungen haben, werden anschliessend mit Methylierungsmitteln der allgemeinen Formel VI

$$H_3C - X \qquad (VI)$$

worin X den Säurerest von starken Sauerstoffsäuren, beispielsweise von Schwefelsäure, Methylschwefelsäure, Fluorsulfonsäure, Trifluormethansulfonsäure, Methansulfonsäure, Benzolsulfonsäure, p-Toluolsulfonsäure, p-Brombenzolsulfonsäure, Phosphorsäure oder auch Halogenid, bevorzugt Chlorid, Bromid, Jodid bedeutet,

zu Pyridiniumsalzen der allgemeinen Formel Va

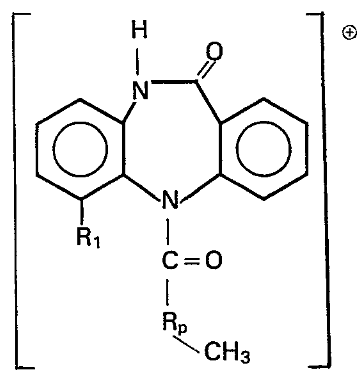

$X^{\ominus}$ (Va)

worin $R_1$, $R_p$ und X die vorstehend genannten Bedeutungen haben, methyliert. Die Methylierung wird in inerten Lösungsmitteln, z.B. chlorierten aliphatischen Kohlenwasserstoffen, wie Methylenchlorid, 1,2-Dichlorethan, offenkettigen oder cyclischen Ethern, wie Diethylether oder Tetrahydrofuran, aromatischen Kohlenwasserstoffen, wie Benzol, Toluol, Xylol oder Dichlorbenzol, bevorzugt jedoch in Dioxan, Acetonitril oder Dimethylformamid und bei Temperaturen zwischen —20 und +130°C, bevorzugt +30 und 100°C durchgeführt.

Anschliessende Reduktion der Pyridiniumsalze Va mit Natrium- oder Kaliumtetrahydridoborat oder Natrium- oder Kaliumalkoxy-, dialkoxy- oder trialkoxyborhydrid in protischen Lösungsmitteln, beispielsweise in Wasser, Methanol, Ethanol, 2-Propanol oder in Gemischen davon bei Temperaturen zwischen —40 und +50°C, bevorzugt bei —5 bis +10°C führt zu den gesuchten Dibenzodiazepinonen der allgemeinen Formel I, in der $R_1$ die eingangs definierten Bedeutungen hat und R einen (1-Methyl-1,2,5,6-tetrahydro-4-pyridinyl)methyl- oder 1-Methyl-1,2,5,6-tetrahydro-4-pyridinyl-Rest bedeutet.

Die Verfahren zur Herstellung der pharmakologisch wirksamen Dibenzodiazepinone der allgemeinen Formel I sind also dadurch gekennzeichnet, dass man Dibenzodiazepinone der allgemeinen Formel II mit Verbindugen der allgemeinen Formel III acyliert oder mit Pyridinalkansäurederivaten der allgemeinen Formel IV umsetzt, anschliessend methyliert und mit Borhydriden oder Alkoxyborhydriden reduziert und jeweils gegebenenfalls anschliessend die so erhaltene Base in ein pharmakologisch verträgliches Säureadditionssalz oder ein erhaltenes Säureadditionssalz in die freie Base oder ein pharmakologisch verträgliches Säureadditionssalz überführt.

c.) Verbindungen der allgemeinen Formel I, in der R einen im heterocyclischen Sechsring gegebenenfalls durch eine oder zwei weitere Methylgruppen

substituierten (1-Methyl-1,2,5,6-tetrahydro-4-pyridinyl)-methyl-, (1-Methyl-1,2,5,6-tetrahydro-4-piperidinyliden)-methyl-, (2,3-Dehydro-8-methyl-8-aza-bicyclo[3,2,1]oct-3-yl)-methyl- oder (8-Methyl-8-aza-bicyclo[3,2,1]oct-3-yliden)-methyl-Rest bedeutet, lassen sich auch dadurch herstellen, dass ein 5-Dialkylphosphonoacetyl-dibenzodiazepinon der allgemeinen Formel VII

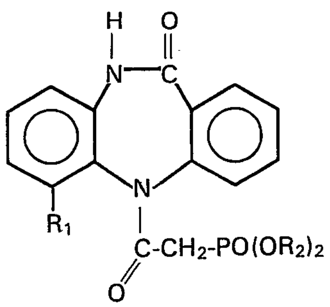

(VII)

in der $R_1$ wie oben definiert ist und $R_2$ eine Alkylgruppe mit 1 bis 10 Kohlenstoffatomen, vorzugsweise eine Ethylgruppe, bedeutet, mit einem Piperidinon bzw. einem Tropinon der allgemeinen Formel VIII bzw. IX

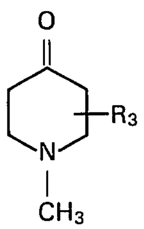

(VIII)

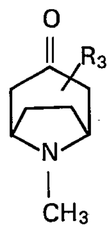

(IX)

in welchen $R_3$ ein Wasserstoffatom oder die Methylgruppe bedeutet, in Gegenwart eines Alkalihydrids oder eines Alkalialkoholats (z.B. Kalium-tert.-butylat) in einem Lösungsmittel bei Temperaturen von 20°C bis zum Siedepunkt des Reaktionsgemisches umgesetzt wird. Als Lösungsmittel dienen Ether, wie Diethylether, Diisopropylether, vorzugsweise aber Tetrahydrofuran. Es entstehen im allgemeinen Gemische, z.B. bei Verwendung von Tropinon neben einer Verbindung der Formel I, in der R den (8-Methyl-8-aza-bicyclo[3,2,1]oct-3-yliden)-methylrest auch eine Verbindung der Formel I, in der R den (2,3-Dehydro-8-methyl-8-aza-bicyclo[3,2,1]-oct-3-yl)-methylrest bedeutet. Derartige Gemische lassen sich meistens leicht in ihre einzelnen Komponenten auftrennen, z.B. mittels Säulenchromatographie.

d.) Zur Herstellung von Verbindungen der allgemeinen Formel I, in der R einen, im heterocyclischen Sechsring gegebenenfalls durch eine oder 2 weitere Methylgruppen substituierten (1-Methyl-4-piperidinyl)-methyl- oder einen endo- oder exo-(8-Methyl-8-aza-bicyclo[3,2,1]oct-3-yl)-methyl-Rest bedeutet, kann man auch ein Dibenzodiazepinon der allgemeinen Formel X

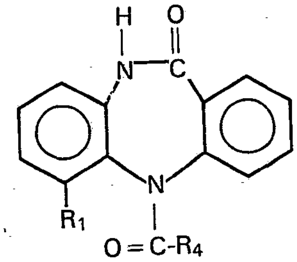

(X)

in der $R_1$ wie oben erwähnt definiert ist und $R_4$ folgende Gruppen

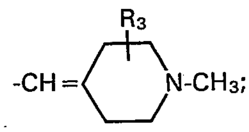;

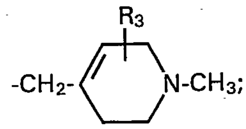;

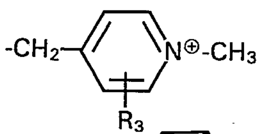 + $X^{\ominus}$;

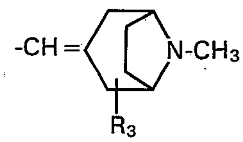

oder 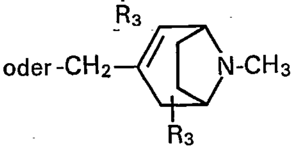 darstellt, wobei in diesen Gruppen $R_3$ ein Wasserstoffatom oder die Methylgruppe darstellt und X ein Halogenatom, vorzugsweise ein Chlor- oder Bromatom ist, katalytisch hydrieren. Die Hydrierung lässt sich beispielsweise mit Platindioxid als Katalysator bei einem Druck zwischen $10^5$ bis $5 . 10^6$ Pa, vorteilhafterweise bei $2 . 10^5$ bis $10^6$ Pa und bei Temperaturen zwischen —20 und +100°C, vorteilhafterweise bei Raumtemperatur, durchführen. Anstelle von Platindioxid lässt sich auch feinverteiltes Palladium auf Kohle oder auch Raney-Nickel oder Raney-Kobalt verwenden. Das zu hydrierende Produkt wird im allgemeinen zuvor aufgelöst, z.B. in einem Alkohol, wie Ethanol.

e.) Sämtliche Verbindungen der allgemeinen Formel I lassen sich aber auch aus einem Dibenzodiazepinon der allgemeinen Formel II

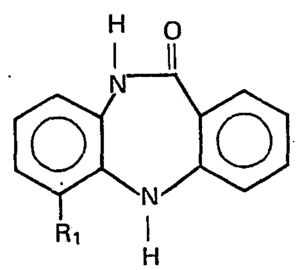

(II)

in der $R_1$ wie oben definiert ist, dadurch herstellen, dass diese Verbindung zuerst mit einem Lithiumalkyl oder Lithiumaryl oder Lithiumamid in ihr Di-Lithiumsalz übergeführt und letzteres anschliessend mit einem Ester der allgemeinen Formel XII

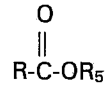

(XII)

in der R die eingangs erwähnten Bedeutungen besitzt und $R_5$ einen Alkylrest mit 1 bis 10 Kohlenstoffatomen, vorzugsweise 1 bis 6 Kohlenstoffatomen, oder einen Aralkylrest mit 7 bis 13 Kohlenstoffatomen, beispielsweise den Phenylmethyl-, Phenylethyl- oder Phenylpropylrest, darstellt, umgesetzt wird.

Die Überführung des Dibenzodiazepinons in sein Dilithiumsalz gelingt insbesondere mit n-Butyllithium, n-Butyllithium in Gegenwart von Tetramethylethylendiamin, tertiär-Butyllithium, Lithiumdiisopropylamid oder Lithiumdicyclohexylamid oder mit Lithiumarylen, wie Lithiumphenyl. Die Überführung in das Lithiumsalz und die weitere Umsetzung mit einer Verbindung der allgemeinen Formel XII erfolgt in einem organischen Lösungsmittel bei Temperaturen zwischen —60 und 0°C, vorzugsweise aber bei —10°C. Als organische Lösungsmittel dienen solche, die für Umsetzungen mit Litiumalkylen oder -arylen gebräuchlich sind; besonders vorteilhaft ist die Verwendung von Tetrahydrofuran oder Ethern, wie Diethylether, von aliphatischen Kohlenwasserstoffen, wie Hexan, oder von Gemischen hiervon, gegebenenfalls auch in Gegenwart von Hexamethylphosphoramid als Cosolvens. Kurze Zeit nach der Zugabe des Lithiumalkyls oder -aryls gibt man die stöchiometrische Menge oder einen leichten Überschuss des Esters der Formel XII hinzu und lässt das Reaktionsgemisch langsam, z.B. innerhalb von 2 Stunden, auf Zimmertemperatur kommen. Die Isolierung des Reaktionsproduktes erfolgt nach an sich üblichen Methoden.

Ein Teil der erfindungsgemässen Dibenzodiazepinone der allgemeinen Formel I enthält ein oder zwei asymmetrische Kohlenstoffatome im Rest R. Diese Verbindungen können deshalb in zwei diastereomeren cis- und trans-Formen oder jeweils als enantiomere (+)- und (—)-Formen auftreten. Die Erfindung umfasst die einzelnen Isomeren ebenso wie ihre Gemische.

Die Trennung der jeweiligen Diastereomeren gelingt aufgrund der unterschiedlichen physikochemischen Eigenschaften, z.B. durch fraktioniertes Umkristallisieren aus geeigneten Lösungsmitteln oder durch chromatographische Verfahren. Jeweils nur ein Diastereomeres wird erhalten, wenn man die oben beschriebene Synthese a) mit nur einem Diastereomeren der allgemeinen Formel III durchführt.

Die Spaltung eventueller Razemate der Verbindungen der allgemeinen Formel I kann nach bekannten Verfahren durchgeführt werden, beispielsweise unter Verwendung einer optisch aktiven Säure, wie (+)- oder (—)-Weinsäure, oder eines Derivats davon, wie (+)- oder (—)-Diacetylweinsäure, (+)- oder (—)-Monomethyltartrat oder (+)-Camphersulfonsäure.

Nach einem üblichen Verfahren zur Isomerentrennung wird das Razemat einer Verbindung der allgemeinen Formel I mit einer der vorstehend beschriebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50 : 50, verwendet. Sodann wird jedes der optisch aktiven Salze in Wasser gelöst, neutralisiert und dadurch die entsprechende freie Verbindung in der (+)- oder (—)-Form erhalten.

Jeweils nur ein Enantiomeres wird auch dadurch erhalten, dass man die oben beschriebene Synthese a) mit nur einem Enantiomeren der allgemeinen Formel III durchführt.

Die als Ausgangsmaterialien erforderlichen 5,10-Dihydro-11H-dibenzo[b,e][1,4]diazepin-11-one der allgemeinen Formel II sind literaturbekannt [z.B. F. Hunziker u.a., Arzneim. Forsch. 13, 324 (1963)].

Verbindungen der allgemeinen Formel III und IV sind bekannt oder können in Analogie zu bekannten Herstellungsverfahren leicht erhalten werden. Beispielsweise erhält man bei der Umsetzung von 4-Hydroxy-1-methyl-4-piperidinessigsäure-natriumsalz mit Thionylchlorid ein Gemisch von 1-Methyl-1,2,5,6-tetrahydro-4-pyridinessigsäurechlorid und (1-Methyl-4-piperidinyliden)-acetylchlorid, das ohne Trennung nach Verfahren a) mit einem Dibenzodiazepinon der allgemeinen Formel II zu einem Gemisch der gesuchten Verbindungen der Formel I umgesetzt werden kann, worin R den (1-Methyl-1,2,5,6-tetrahydro-4-pyridinyl)-methyl und (1-Methyl-4-piperidinyliden)-methyl-Rest bedeutet. Dieses Gemisch von Doppelbindungsisomeren kann anschliessend gewünschtenfalls nach üblichen Verfahren, beispielsweise durch fraktionierte Kristallisation, Säulenchromatographie oder Hochdruckflüssigkeitschromatographie, in seine Bestandteile zerlegt werden.

Weiterhin erhält man Tropan-3α-essigsäure und Tropan-3β-essigsäure jeweils frei von dem anderen Isomeren nach W. Schneider u.a., Arch. Pharm. 308, 365-375 (1975) bzw. Ch. L. Zirkle u.a., J. Org. Chem. 27, 1279-1285 (1962). Als reaktive Säurederivate dieser Verbindungen seien die Säurechloride erwähnt, die man in üblicher Weise aus den genannten Carbonsäuren durch Überführung ins Kaliumsalz und anschliessende Behandlung mit gereinigtem Thionylchlorid herstellen kann.

Gegebenenfalls methyl- oder dimethylsubstituierte 4-Pyridinessigsäuren oder Isonicotinsäuren sind käuflich oder können in Analogie zu einer bzw. durch eine Arndt-Eistert-Reaktion über die von D. Jerchel u.a., Liebigs Ann. Chem. 613, 153-170 (1958) bzw. von R. Lukes u.a., Collect. Czechoslov. Chem. Commun. 23, 1083-1089 (1958); 27, 2220-2222 (1962) beschriebenen substituierten Isonicotinsäuren synthetisiert werden. Als reaktive Säurederivate kommen beispielsweise die Säurechloridhydrochloride in Betracht, die nach oder in Analogie zu H. Leditschke, Arch. Pharm. 295, 328 (1962) erhalten werden können.

Wie bereits oben erwähnt, weisen die neuen Verbindungen der allgemeinen Formel I wertvolle pharmakologische Eigenschaften auf; insbesondere besitzen sie antiulcerogene und magensäure-sekretionshemmende Wirkungen, sowie günstige Effekte auf verschiedene andere Erkrankungen des Magen-Darm-Traktes, unter denen das Colon irritable besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, dass die erfindungsgemässen Verbindungen diesbezüglich überraschend günstige Relationen aufweisen.

*Untersuchung auf Selektivität der antimuscarinischen Wirkung*

*Ziel:*

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

*Methodik:*

Verwendet wurden 10 weibliche Albino-Ratten [Stamm Crl: COBS-CD (SD) BR] pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100% der Tiere ausgelöst hatte.

| | |
|---|---|
| Magenschleimhaut-Läsionen | 0,62 mg/kg i.v. |
| Speichelsekretion: | 0,083 mg/kg i.v. |

Jede antimuscarinische Substanz wurde in gleichmässig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorininduzierten Speichelsekretion wurde blind durchgeführt, d.h. der Untersucher wusste nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. 96, 99, 1949) ermittelt.

Die Effekte auf Schleimhaut-Läsionen des Magens wurden folgendermassen bewertet:

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. $ED_{50}$- bzw. $ED_{70}$-Werte wurden nach der Methode von LITCHFIELD und WILCOXON (s.o.) ermittelt.

*Mydriasis*

Der Effekt von Testsubstanzen auf die Pupillenweite von Ratten wurde folgendermassen untersucht:

Die Substanzen wurden an Gruppen von jeweils 10 Tieren in wenigstens 3 gleichmässig abgestuften Dosierungen intravenös appliziert. Die Pupillenweite wurde anschliessend 10 Minuten lang auf evtl. Veränderungen (Auftreten von Mydriasis oder Miosis) beobachtet, und zwar wiederum blind, d.h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s.o.) bestimmt.

2. *Bindungsstudien an muscarinischen Rezeptoren: Bestimmung des $IC_{50}$-Wertes*

Als Organspender dienten männliche Sprague-Dawley-Ratten mit 180-220 g Körpergewicht. Nach Entnahme von Herz, Magen und Grosshirnrinde wurden alle weiteren Schritte im eiskalten Hepes-HCl-Puffer (pH 7,4; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschliessend in einem Potter homogenisiert.

Für den Bindungstest wurden die Organhomogenate in folgender Weise verdünnt:

| | |
|---|---|
| Glatter Muskel Magenfundus | 1 : 100 |
| Gesamtherz | 1 : 250 |
| Grosshirnrinde | 1 : 3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50% gehemmt wurde.

Nach den vorstehenden Angaben wurden beispielsweise die folgenden Verbindungen untersucht:

A = 5,10-Dihydro-5-[(1-methyl-1,2,5,6-tetrahydro-4-pyridinyl)carbonyl]-11H-dibenzo-[b,e][1,4]diazepin-11-on

B = 6-Chlor-5,10-dihydro-5-[(1-methyl-4-piperidinyl)acetyl]-11H-dibenzo[b,e][1,4]diazepin-11-on

C = endo-5,10-Dihydro-5-[(8-methyl-8-azabicyclo[3,2,1]oct-3-yl)acetyl]-11H-dibenzo-[b,e][1,4]diazepin-11-on

*Ergebnisse:*

| Substanz | Rezeptoren-Bindung-Tests $IC_{50}$ [n Mol $1^{-1}$] Cortex | Glatter Muskel Magenfundus | Herz | Oxotremorin-Tests [µg/kg] i.v. Antiulcerogene Wirkung $ED_{50}$ | $ED_{70}$ | Salivations-Hemmung $ED_{50}$ | Mydriasis $ED_{50}$ [µg/kg] i.v. |
|---|---|---|---|---|---|---|---|
| A | 30 | 100 | 250 | 3,1 | 6,5 | 80 | 36,6 |
| B | 28 | 170 | 200 | 4,4 | 8,4 | 230 | 138 |
| C | 15 | 200 | 55 | 1,1 | 3,8 | 105 | 54 |

Die Angaben in der vorstehenden Tabelle beweisen, dass die genannten Verbindungen generell eine hohe Affinität zu muscarinischen Rezeptoren besitzen. Darüberhinaus kann man den Daten entnehmen, dass die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Grosshirnrinde gegenüber solchen aus der glatten Muskulatur von Magen und Herz.

Aus den pharmakologischen Daten der vorstehenden Tabelle ergibt sich — in völliger Übereinstimmung mit den Rezeptor-Bindungs-Studien —, dass die Entstehung Oxotremorin-induzierter Magenschleim-haut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. «F.» bedeutet «Schmelzpunkt», «Z.» bedeutet «Zersetzung».

*Beispiel 1*

*5,10-Dihydro-5-[(1-methyl-1,2,5,6-tetrahydro--4-pyridinyl)acetyl]-11H-dibenzo[b,e][1,4]diazepin-11-on*

Unter äusserer Kühlung und unter Einhaltung einer Reaktionstemperatur von +15°C wurde zu einer Suspension von 14,0 g (0,072 Mol) 1-Methyl-1,2,-5,6-tetrahydro-4-pyridinessigsäure-Kaliumsalz in 150 ml wasserfreiem Chloroform 5,8 ml (0,081 Mol) Thionylchlorid, gelöst in 20 ml Chloroform, zugetropft. Man rührte 20 Minuten nach, engte die Mischung im Vakuum zur Trockne ein und trug den verbleibenden Rückstand in eine Aufschlämmung von 8,4 g (0,04 Mol) 5,10-Dihydro-11H-dibenzo[b,e]-[1,4]diazepin-11-on in einem Lösungsmittelgemisch aus 300 ml absolutem Dioxan und 20 ml wasserfreiem Pyridin ein. Unter kräftigem Rühren wurde die Mischung 2 Stunden auf 80°C erhitzt. Nach dem Erkalten wurde filtriert, der Filterrückstand in Wasser aufgenommen. Man stellte mit festem Natriumcarbonat alkalisch und extrahierte die wässrige Phase erschöpfend mit Chloroform. Die vereinigten Chloroformauszüge wurden getrocknet und im Vakuum vom Lösungsmittel befreit. Der Rückstand wurde an Kieselgel unter Verwendung von Essigsäureethylester/Methanol (Volumenverhältnis 9 : 1) zum Eluieren säulenchromatographisch gereinigt. Die Substanz wurde als amorphes Produkt isoliert. Ausbeute: 3,5 g (25% der Theorie).

$C_{21}H_{21}N_3O_2$ (347,42)
Ber.: C 72,6 H 6,09 N 12,09
Gef.: C 71,9 H 6,31 N 12,14

IR (KBr): C = O 1660, Schulter 1680 $cm^{-1}$

UV (Ethanol): Schulter bei $\lambda$ = 270 nm
(Ethanol/KOH): $\lambda_{max}$ 290 nm (E = 0,08), Schulter bei $\lambda$ = 260 nm (E = 0,105)

$^1$H-NMR ($d_6$-DMSO, 80 MH): $\delta$ = 7,1-7,9 (8H-m, aromat. H); 5,02 breit, 1H, olef. H); 2,95 breit (2H, C = C-$CH_2$-N); 2,68 (2H, N-$CH_2$); 2,15 bis 2,5 (2H-m; CO-$CH_2$); 2,18 (3H-s; $NCH_3$); 1,84 breit (2H; c=c-$CH_2$); 12,00 breit (1H-s; austauschbarer H).

*Beispiel 2*

*5,10-Dihydro-5-[(1-methyl-1,2,5,6-tetrahydro--4-pyridinyl)acetyl]-11H-dibenzo[b,e][1,4]diazepin-11-on* und *5,10-Dihydro-5-[(1-methyl-4-piperidinyliden)acetyl]-11H-dibenzo[b,e][1,4]diazepin-11-on*

In eine Suspension von 16,8 g (0,08 Mol) des Kaliumsalzes der 4-Hydroxyl-1-methyl-4-piperdinessigsäure in 300 ml Chloroform wurde unter äusserer Kühlung und unter Einhaltung einer Reaktionstemperatur von 15°C eine Lösung von 14,2 g (0,12 Mol) Thionylchlorid in 50 ml Chloroform eingetropft. Nach 20minütigem Rühren wurde im Vakuum eingedampft, der Rückstand anschliessend in eine Aufschlämmung von 8,4 g (0,04 Mol) 5,10-Dihydro--11H-dibenzo[b,e][1,4]diazepin-11-on in einem Gemisch aus 400 ml wassefreiem Dioxan und 20 ml trockenem Pyridin eingetragen. Unter kräftigem Rühren wurde 2 Stunden auf 80°C erhitzt. Nach dem Erkalten wurde der Niederschlag abfiltriert und in Wasser aufgenommen. Die wässrige Lösung wurde mit festem Natriumcarbonat alkalisiert und anschliessend erchöpfend mit Chloroform ausgezogen. Die vereinigten Extrakte wurden getrocknet und im Vakuum eingedampft, der Rückstand anschliessend an Aluminiumoxid (Aktivitätsstufe 1) unter Verwendung von Essigsäureethylester/Methanol (Volumenverhältnis 99 : 1) zum Eluieren säulenchromatographiert. Man erhielt 2,5 g (18% der Theorie) an 5,10-Dihydro-5-[(1-methyl-1,2,5,6-tetrahydro-4--pyridinyl)acetyl]-11H-dibenzo[b,e][1,4]diazepin--11-on, nach Dünnschichtchromatogramm, IR- und NMR-Spektren völlig identisch mit einem nach Beispiel 1 hergestellten Präparat, sowie 0,2 g (1,4% der Theorie) an 5,10-Dihydro-5-[(1-methyl-4-piperidinyliden)acetyl]-11H-dibenzo[b,e][1,4]diazepin-11--on vom F. 200-201 °C.

Entsprechend wurden erhalten:

*6-Chlor-5,10-dihydro-5-[(1-methyl-1,2,5,6-tetrahydro-4-pyridinyl)acetyl]-11H-dibenzo[b,e][1,4]diazepin-11-on* und

*6-Chlor-5,10-dihydro-5-[(1-methyl-4-piperidinyliden)acetyl]-11H-dibenzo[b,e][1,4]diazepin-11-on*

durch Umsetzung von 4-Hydroxy-1-methyl-4-piperidinessigsäure und 6-Chlor-5,10-dihydro-11H-dibenzo[b,e][1,4]diazepin-11-on mit einer Ausbeute von 31% der Theorie. Das 6-Chlor-5,10-dihydro-5--[(1-methyl-1,2,5,6-tetrahydro-4-pyridinyl)-acetyl]--11H-dibenzo[b,e][1,4]diazepin-11-on entstand als amorphes Pulver vom Zersetzungspunkt 180 °C;

$C_{21}H_{20}ClN_3O_2$ (381,87)
Ber.: C 66,05 H 5,28 Cl 9,28 N 11,01
Gef.: C 66,12 H 5,07 Cl 9,00 N 11,20

$^1$H-NMR ($CDCl_3$, 80 MHz): δ = 7,9 (1H-m, aromat. H in 1-Stellung); 6,9-7,6 (6H-m, aromat. H); 4,9 und 5,15 (1H, olef. H); 2,8-3,05 (4H-m; $2CH_2$); 2,4-2,65 (2H-m, $CH_2$); 2,3 und 2,35 (3H, $N-CH_3$); 2,05 (2H, $CH_2$);

Das 6-Chlor-5,10-dihydro-5-[(1-methyl-4-piperidinyliden)acetyl]-11H-dibenzo[b,e][1,4]diazepin--11-on entstand daneben mit einer Ausbeute von 10% als amorphes Pulver von Zersetzungspunkt 155 °C.

$C_{21}H_{20}ClN_3O_2$ (381,87)
Ber.: C 66,05 H 5,28 Cl 9,28 N 11,01
Gef.: C 66,15 H 5,32 Cl 9,09 N 11,03

$^1$H-NMR ($CDCl_3$): δ = 7,9-8,1 (1H-m; aromat. H in 1-Stellung); 6,9-7,65 (6H-m, aromat. H); 5,4 und 5,55 (1H, olef. H); 2,8-3,05 (2H-m, $CH_2$); 1,95-2,55 (6H-m; $3CH_2$); 2,2 (3H, $N-CH_3$).

*Beispiel 3*

*endo-5,10-Dihydro-5-[(8-methyl-8-azabicyclo-[3,2,1]oct-3-yl)acetyl]-11H-dibenzo[b,e][1,4]diazepin-11-on*

Hergestellt analog Beispiel 2 aus Tropan-3α--essigsäure-Kaliumsalz und 5,10-Dihydro-11H-dibenzo[b,e][1,4]diazepin-11-on in einer Ausbeute von 33% der Theorie. F. 226-227 °C (Essigsäureethylester).

Entsprechend wurde erhalten:

*endo-6-Chlor-5,10-dihydro-5-[(8-methyl-8-azabicyclo[3,2,1]oct-3-yl)acetyl-11H-dibenzo[b,e]-[1,4]diazepin-11-on*

durch Umsetzung von Tropan-3α-essigsäure-Kaliumsalz und 6-Chlor-5,10-dihydro-11H-dibenzo-[b,e][1,4]diazepin-11-on in einer Ausbeute von 27% der Theorie als amorphes Pulver;

$C_{23}H_{24}ClN_3O_2$ (409,93)
Ber.: C 67,39 H 5,90 Cl 8,65 N 10,25
Gef.: C 67,42 H 5,85 Cl 8,60 N 10,08

$^1$H-NMR ($CDCl_3$): δ = 7,8-8,0 (1H-m; aromat. H in 1-Stellung); 7,0-7,6 (6H-m, aromat. H); 1,3-3,2 (16H-m, CH, $CH_2$ und $N-CH_3$ bei 2,2)

IR (KBr): C=O 1665, Schulter 1675 $cm^{-1}$.

*Beispiel 4*

*5,10-Dihydro-5-[(1-methyl-4-piperidinyl)acetyl]--11H-dibenzo[b,e][1,4]diazepin-11-on*

Hergestellt analog Beispiel 2 aus 1-Methyl-4--piperidinessigsäure-Kaliumsalz und 5,10-Dihydro--11H-dibenzo[b,e][1,4]diazepin-11-on in einer Ausbeute von 26% der Theorie. Wachsartige Substanz, F. ≥ 95 °C.

$C_{21}H_{23}N_3O_2$ (349,43)
Ber.: C 72,18 H 6,63 N 12,03
Gef.: C 71,72 H 7,07 N 14,90

MS: $\frac{m}{e}$ = 349 (210; 140; 96)

IR (($CH_2Cl_2$): NH 3375, CO 1660 und 1676 $cm^{-1}$

UV (Ethanol): Schulter bei λ = 270 nm
UV (Ethanol/KOH): $λ_{max}$ 290 nm (E = 0,095); Schulter bei λ 256 nm (E = 0,12)

$^1$H-NMR ($CDCl_3/D_2O$; 80 MHz): δ = 7,7-8,0 (1H-m; aromat. H); 7,0-7,8 (7H-m; ar. H); 2,5-2,9 (2H-m); 0,8-2,4 (12H-m), davon bei δ = 2,12 (3H-s; $N-CH_3$)

Entsprechend erhielt man:

*5,10-Dihydro-5-[(1,3-dimethyl-4-piperidinyl]acetyl]-11H-dibenzo[b,e][1,4]diazepin-11-on*

aus 5,10-Dihydro-11H-dibenzo[b,e][1,4]diazepin--11-on und 1,3-Dimethyl-4-piperidinessigsäure-Kaliumsalz in einer Ausbeute von 45% der Theorie, F. 175 °C;

MS: $\frac{m}{e}$ = 363;

*5,10-Dihydro-5-[(1,2-dimethyl-4-piperidinyl)acetyl]-11H-dibenzo[b,e][1,4]diazepin-11-on*

aus 5,10-Dihydro-11H-dibenzo[b,e][1,4]diazepin--11-on und 1,2-Dimethyl-4-piperidinessigsäure-Kaliumsalz in einer Ausbeute von 38% der Theorie;

MS: $\frac{m}{e}$ = 363;

IR (KBr): C=O 1665 $cm^{-1}$.

*exo-5,10-Dihydro-5-[(8-methyl-8-azabicyclo-[3,2,1]oct-3-yl)acetyl]-11H-dibenzo[b,e][1,4]diazepin-11-on*

aus 5,10-Dihydro-11H-dibenzo[b,e][1,4]diazepin-11-on und Tropan-3β-essigsäure-Kaliumsalz in einer Ausbeute von 48% der Theorie, F. 148-150°C; und

*exo-6-Chlor-5,10-dihydro-5-[(8-methyl-8-azabicyclo[3,2,1]oct-3-yl)acetyl]-11H-dibenzo[b,e][1,4]-diazepin-11-on*

aus 6-Chlor-5,10-dihydro-11H-dibenzo[b,e][1,4]-diazepin-11-on und Tropan-3β-essigsäure-Kaliumsalz in einer Ausbeute von 51% der Theorie;

$C_{23}H_{24}ClN_3O_2$ (409,93)

| | | | | |
|---|---|---|---|---|
| Ber.: | C 67,39 | H 5,90 | Cl 8,65 | N 10,25 |
| Gef.: | C 67,30 | H 5,82 | Cl 8,91 | N 10,09 |

IR (KBr): C=O 1665 $cm^{-1}$.

*Beispiel 5*

*6-Chlor-5,10-dihydro-5-[(1-methyl-4-piperidinyl)-acetyl]-11H-dibenzo[b,e][1,4]diazepin-11-on*

Zu einer Suspension von 1,8 g (7 mMol) 6-Chlor-5,10-dihydro-11H-dibenzo[b,e][1,4]diazepin-11-on und 7,0 g (28 mMol) 1-Methyl-4-piperidinessigsäurechlorid-hydrochlorid in 200 ml Dioxan tropfte man bei Raumtemperatur innerhalb von 20 Minuten eine Mischung von 1,7 g (21 mMol) Pyridin und 30 ml Dioxan. Es wurde 7 Stunden bei Zimmertemperatur gerührt und anschliessend das Reaktionsgemisch filtriert. Die festen Anteile wurden zwischen Chloroform und einer wässrigen Kaliumcarbonat-Lösung verteilt. Die organische Phase wurde über Magnesiumsulfat getrocknet, mit Tierkohle behandelt, filtriert und eingedampft. Der Rückstand wurde an Kieselgel (Macherey & Nagel MN 60; Elutionsmittel: Chloroform/Essigsäureethylester/Methanol im Volumenverhältnis 1 : 1 : 1) säulenchromatographisch gereinigt und ergab nach Anreiben mit Cyclohexan 0,85 g (32% der Theorie) an 6-Chlor-5,10-dihydro-5-[(1-methyl-4-piperidinyl)acetyl]-11H-dibenzo-[b,e][1,4]diazepin-11-on.

$C_{21}H_{22}ClN_3O_2$ (383,88)

| | | | | |
|---|---|---|---|---|
| Ber.: | C 65,71 | H 5,78 | Cl 9,24 | N 10,95 |
| Gef.: | C 65,90 | H 6,02 | Cl 8,78 | N 10,90 |

$^1$H-NMR ($CDCl_3$; 80 MHz): $\delta$ = 7,8-8,0 (1H-m; 1 ar. H in 1-Stellung); 7,0-7,6 (6H-m; ar. H); 2,2 (3H-s; $N-CH_3$); 2,6-2,9 und 1,1-2,3 (11H-m; aliph. H).

IR ($CH_2Cl_2$): NH 3400 und 3200 $cm^{-1}$; C=O 1670 und Schulter 1690 $cm^{-1}$

UV (Ethanol): $\lambda$ = 270 nm
(Ethanol/KOH): $\lambda$ = 259 nm und 289 nm.

6-Chlor-5,10-dihydro-5-[(1-methyl-4-piperidinyl)acetyl]-11H-dibenzo[b,e][1,4]diazepin-11-on kristallisiert aus Essigester mit 1/2 Mol Essigester: F. 147°C.

*Beispiel 6*

*5,10-Dihydro-5-[(1-methyl-1,2,5,6-tetrahydro-4-pyridinyl)carbonyl]-11H-dibenzo[b,e][1,4]diazepin-11-on-hemihydrat*

Die Mischung aus 10,65 g (0,051 Mol) 5,10-Dihydro-11H-dibenzo[b,e][1,4]diazepin-11-on, 19,6 g (0,10 Mol) 1-Methyl-1,2,5,6-tetrahydroisonicotinsäurechlorid-hydrochlorid, 20,7 g (0,15 Mol) Kaliumcarbonat und 200 ml wasserfreiem Toluol wurde unter gutem Rühren 24 Stunden unter Rückfluss gekocht. Nach dem Erkalten wurde in 500 ml Eiswasser eingerührt, die organische Schicht abgetrennt und die wässrige erschöpfend mit Essigsäureethylester extrahiert. Die vereinigten organischen Phasen wurden über Natriumsulfat getrocknet, dann im Vakuum eingedampft. Der Rückstand wurde einmal an Kieselgel 0,1 bis 0,4 mm Korngrösse unter Verwendung von 1,2-Dichlorethan/Methanol (Volumenverhältnis 9 : 1) und einmal an Kieselgel 0,05 bis 0,2 mm Korngrösse unter Verwendung von 1,2-Dichlorethan/Methanol (Volumenverhältnis 95 : 5) als Eluentien säulenchromatographisch gereinigt. Aufarbeitung der geeigneten Fraktionen in der üblichen Weise ergab farblose Kristalle von F. 233-234°C (Diisopropylether/Essigsäureethylester).

Ausbeute: 2,4 g (14% der Theorie).

*Beispiel 7*

*5,10-Dihydro-5-[(1-methyl-1,2,5,6-tetrahydro-4-pyridinyl)carbonyl]-11H-dibenzo[b,e][1,4]diazepin-11-on-hemihydrat*

a) *5,10-Dihydro-5-[(4-pyridinyl)carbonyl]-11H-dibenzo[b,e][1,4]diazepin-11-on*

Ein Gemisch von 21,0 g (0,1 Mol) 5,10-Dihydro-11H-dibenzo[b,e][1,4]diazepin-11-on, 23,0 g (0,129 Mol) Isonicotinsäurechlorid-hydrochlorid, 21 ml (0,26 Mol) Pyridin und 300 ml wasserfreiem Dioxan wurde 3 Stunden unter Rückfluss gekocht. Der erkaltete Ansatz wurde in 1 *l* Eiswasser eingegossen, mit konz. Salzsäure angesäuert und zweimal mit je 200 ml Dichlormethan ausgeschüttelt. Die wässrige Phase wurde mit Natriumcarbonat alkalisch gestellt und erschöpfend mit Methylenchlorid extrahiert. Die vereinigten Methylenchloridextrakte wurden mit Wasser gewaschen, über Natriumsulfat getrocknet und im Vakuum vom Lösungsmittel befreit. Der Rückstand wurde aus heissem Methanol umkristallisiert. Nach dem Waschen mit Ether schmolzen die beigefarbenen Kristalle bei 277-279°C.

Ausbeute 16,0 g (51% der Theorie).

b) *5,10-Dihydro-5-[(4-pyridinyl)carbonyl]-11H-dibenzo[b,e][1,4]diazepin-11-on-methoiodid*

6,3 g (0,02 Mol) 5,10-Dihydro-5-](4-pyridinyl)-carbonyl]-11H-dibenzo[b,e][1,4]diazepin-11-on wurden in 100 ml wasserfreiem Dimethylformamid gelöst und nach Zugabe von 3,2 g (0,0227 Mol) Jodmethan über Nacht bei Zimmertemperatur gerührt. Man engte im Vakuum auf ein Drittel des ursprünglichen Volumens ein, fügte dann ein Gemisch aus gleichen Volumenteilen Methanol und Ether zu, bis das entstandene Salz auszukristallisieren begann. Nach zweistündigem Stehenlassen bei Zimmertemperatur saugte man ab und wusch den Niederschlag gründlich mit Ether durch. Man erhielt 7,2 g (79% der Theorie) an gelben Kristallen vom F. 297°C (Zersetzung).

c) *5,10-Dihydro-5-[(1-methyl-1,2,5,6-tetrahydro-4-pyridinyl)carbonyl]-11H-dibenzo[b,e][1,4]-diazepin-11-on-hemihydrat*

2,3 g (0,005 Mol 5,10-Dihydro-5-[(4-pyridinyl)-carbonyl]-11H-dibenzo[b,e][1,4]diazepin-11-on-methoiodid wurden in 200 ml Methanol suspendiert und bei 0°C portionsweise mit 0,25 g (0,006 Mol) Natriumborhydrid versetzt. Es wurde noch 1 Stunde im Eisbad nachgerührt. Dann wurde die Mischung in 1 *l* Eiswasser eingerührt und mit Methylenchlorid erschöpfend extrahiert. Die vereinigten Extrakte wurden mit Wasser gewaschen, über Natriumsulfat getrocknet und im Vakuum eingeengt. Nach dem Umkristallisieren aus Diisopropylether/Methanol schmolzen die farblosen Kristalle bei 233-234°C.

Ausbeute: 0,9 g (53% der Theorie).

Das Produkt war nach Dünnschichtchromatogramm, IR- und NMR-Spektrum mit einem nach Beispiel 6 erhaltenen Präparat identisch.

*Beispiel 8*

*5,10-Dihydro-5-[(1,2,6-trimethyl-1,2,5,6-tetrahydro-4-pyridinyl)carbonyl]-11H-dibenzo[b,e][1,4]-diazepin-11-on*

a) *5,10-Dihydro-5-[(2,6-dimethyl-4-pyridinyl)-carbonyl]-11H-dibenzo[b,e][1,4]diazepin-11-on*

Hergestellt analog Beispiel 7a) aus 5,10-Dihydro-11H-dibenzo[b,e][1,4]diazepin-11-on und 2,5-Dimethylisonicotinsäurechlorid-hydrochlorid (F. 135 bis 138°C) in einer Ausbeute von 54% der Theorie. Hellgelbe Kristalle vom F. 273-275°C (Methanol unter Verwendung von Aktivkohle).

b) *5,10-Dihydro-5-[(2,6-dimethyl-4-pyridinyl)-carbonyl]-11H-dibenzo[b,e][1,4]diazepin-11-on-methoiodid*

Hergestellt analog Beispiel 7b) aus 5,10-Dihydro-5-[(2,6-dimethyl-4-pyridinyl)carbonyl]-11H-dibenzo[b,e][1,4]diazepin-11-on und Jodmethan in einer Ausbeute von 62% der Theorie. Die gelben Kristalle schmolzen unter Zersetzung bei 298-300°C.

c) *5,10-Dihydro-5-[(1,2,6-trimethyl-1,2,5,6-tetrahydro-4-pyridinyl]-11H-dibenzo[b,e][1,4]diazepin-11-on*

Hergestellt analog Beispiel 7c) aus 5,10-Dihydro-5-[(2,6-dimethyl-4-pyridinyl)carbonyl]-11H-dibenzo[b,e][1,4]diazepin-11-on-methoiodid und Natriumborhydrid in Methanol in einer Ausbeute von 25% der Theorie. Nach säulenchromatographischer Reinigung an Kieselgel unter Verwendung von Dichlormethan/Methanol (Volumenverhältnis 9 : 1) zum Eluieren und Umkristallisieren aus Diisopropylether/Methanol schmolzen die leicht beige gefärbten Kristalle bei 124-125°C.

Entsprechend erhielt man:

*5,10-Dihydro-5-[(1,3-dimethyl-1,2,5,6-tetrahydro-4-pyridinyl)carbonyl]-11H-dibenzo[b,e][1,4]diazepin-11-on* und
*5,10-Dihydro-5-[(1,5-dimethyl-1,2,5,6-tetrahydro-4-pyridinyl)carbonyl]-11H-dibenzo[b,e][1,4]diazepin-11-on*

durch Umsetzung von 5,10-Dihydro-11H-dibenzo-[b,e][1,4]diazepin-11-on mit 3-Methylisonicotinsäurechlorid (F. 155-156°C) über 5,10-Dihydro-5-[(3-methyl-4-pyridinyl)carbonyl]-11H-dibenzo[b,e]-[1,4]diazepin-11-on vom F. 223-224°C (Diisopropylether/Methanol und 5,10-Dihydro-5-[(3-methyl-4-pyridinyl)carbonyl]-11H-dibenzo[b,e][1,4]diazepin-11-on-methoiodid;

*5,10-Dihydro-5-[(1,2-dimethyl-1,2,5,6-tetrahydro-4-pyridinyl)carbonyl]-11H-dibenzo[b,e][1,4]diazepin-11-on* und das
*5,10-Dihydro-5-[(1,6-dimethyl-1,2,5,6-tetrahydro-4-pyridinyl)carbonyl]-11H-dibenzo[b,e][1,4]diazepin-11-on;*

aus 5,10-Dihydro-11H-dibenzo[b,e][1,4]diazepin-11-on und 2-Methylisonicotinsäurechlorid-hydrochlorid über 5,10-Dihydro-5-[(2-methyl-4-pyridinyl)carbonyl]-11H-dibenzo[b,e][1,4]diazepin-11-on und 5,10-Dihydro-5-[(2-methyl-4-pyridinyl)carbonyl]-11H-dibenzo[b,e][1,4]diazepin-11-on-methoiodid.

*6-Chlor-5,10-dihydro-5-[(1-methyl-1,2,5,6-tetrahydro-4-pyridinyl)carbonyl]-11H-dibenzo[b,e][1,4]-diazepin-11-on*

aus 6-Chlor-5,10-dihydro-11H-dibenzo[b,e][1,4]-diazepin-11-on und Isonicotinsäurechlorid-hydrochlorid über 6-Chlor-5,10-dihydro-5-[(4-pyridinyl)-carbony]-11H-dibenzo[b,e][1,4]diazepin-11-on und 6-Chlor-5,10-dihydro-5-[(4-pyridinyl)carbonyl]-11H-dibenzo[b,e][1,4]diazepin-11-on-methoiodid; F. 296°C (aus Methanol) in einer Gesamtausbeute von 53% der Theorie, als amorphe Substanz durch Behandeln mit Methanol und Diisopropylether;

IR-Spektrum ($CH_2Cl_2$): NH 3380 $cm^{-1}$; C=O 1675 $cm^{-1}$,

*5,10-Dihydro-5-[(1-methyl-1,2,5,6-tetrahydro-4-pyridinyl)acetyl]-11H-dibenzo[b,e][1,4]diazepin-11-on*

aus 5,10-Dihydro-11H-dibenzo[b,e][1,4]diazepin-11-on und 4-Pyridinessigsäurechlorid-hydrochlorid über 5,10-Dihydro-5-[(4-pyridinyl)acetyl]-11H-dibenzo[b,e][1,4]diazepin-11-on und 5,10-Dihydro-5-[(4-pyridinyl)acetyl]-11H-dibenzo[b,e][1,4]diazepin-11-on-methoiodid.

*Beispiel 9*

*5,10-Dihydro-5-[(1-methyl-1,2,5,6-tetrahydro-4-pyridinyl)acetyl]-11H-dibenzo[b,e][1,4]diazepin-11-on*

Man erwärmte ein Gemisch von 0,97 g (6,25 Mol) 1-Methyl-1,2,5,6-tetrahydro-4-pyridinessigsäure und 0,20 g (6,25 mMol) 75 proz. Natriumhydrid (in Paraffinöl) in 16 ml Dimethylformamid bei 50-80°C so lange, bis die Wasserstoffentwicklung beendet war (2 bis 3 Stunden). Zu dem so hergestellten Natriumsalz der Säure fügte man 1,312 g (6,24 mMol) 5,10-Dihydro-11H-dibenzo[b,e][1,4]diazepin-11-on und tropfte bei —10°C 0,99 g 98 proz. Phosphoroxychlorid innerhalb von 10 Minuten zu. Man rührte 4 Stunden bei —10°C, 4 Stunden bei 0°C und 20 Stunden bei Zimmertemperatur. Man rührte

den Ansatz in 200 g gestossenes Eis ein, stellte mit Natronlauge auf pH 3,5, schüttelte mit Methylenchlorid einmal aus, stellte dann die wässrige Phase auf pH 9 und zog erchöpfend mit Methylenchlorid aus. Man wusch die organische Phase mit Wasser, trocknete sie über Natriumsulfat und engte sie im Vakuum ein. Man erhielt nach säulenchromatographischer Reinigung an Kieselgel (Essigsäureethylester/Methanol im Volumenverhältnis 9 : 1 zum Eluieren) 0,63 g (29% der Theorie) eines farblosen amorphen Produkts, des nach Dünnschichtchromatogramm, Elementaranalyse und IR-Spektrum identisch war mit einem nach Beispiel 1 hergestellten Präparat.

Entsprechend erhält man:

endo-5,10-Dihydro-5-[(8-methyl-8-azabicyclo-[3,2,1]oct-3-yl)acetyl]-11H-dibenzo[b,e][1,4]diazepin-11-on

exo-5,10-Dihydro-5-[(8-methyl-8-azabicyclo-[3,2,1]oct-3-yl)acetyl]-11H-dibenzo[b,e][1,4]diazepin-11-on

5,10-Dihydro-5-[(1-methyl-4-piperidinyl)acetyl]-11H-dibenzo[b,e][1,4]diazepin-11-on

6-Chlor-5,10-dihydro-5-[(1-methyl-4-piperidinyl)-acetyl]-11H-dibenzo[b,e][1,4]diazepin-11-on

5,10-Dihydro-5-[(1-methyl-1,2,5,6-tetrahydro-4-pyridinyl)carbonyl]-11H-dibenzo[b,e][1,4]diazepin-11-on-hemihydrat.

*Beispiel 10*

*5,10-Dihydro-5-[(1-methyl-1,2,5,6-tetrahydro-4-pyridinyl)acetyl]-11H-dibenzo[b,e][1,4]diazepin-11-on*

Zu einer Suspension von 1,552 g (10 mMol) 1-Methyl-1,2,5,6-tetrahydro-4-pyridinessigsäure in 20 ml wasserfreiem Tetrahydrofuran wurden bei 0°C 1,1 g (10,14 mMol) Chlorkohlensäureethylester zugetropft. Man fügte 2,10 g (10 mMol) 5,10-Dihydro-11H-dibenzo[b,e][1,4]diazepin-11-on zu dem entstandenen Gemisch, rührte noch 1 Stunde bei 0°C und anschliessend 4 Stunden bei Zimmertemperatur. Dann rührte man unter äusserer Kühlung mit Eis in 160 ml 2N Natronlauge ein, extrahierte erschöpfend mit Dichlormethan und dampfte die organische Phase im Vakuum zur Trockne ein. Nach säulenchromatographischer Reinigung an Kieselgel unter Verwendung von Essigsäureethylester/Methanol im Volumenverhältnis 9 : 1 zum Eluieren erhielt man 0,87 g (25% der Theorie) eines farblosen, amorphen Produkts, das nach Dünnschichtchromatogramm, IR- und NMR-Spektrum mit einem nach Beispiel 1 synthetisierten Präparat völlig identisch war.

Analog erhielt man

aus 5,10-Dihydro-11H-dibenzo[b,e][1,4]diazepin-11-on und 1-Methyl-1,2,5,6-tetrahydro-isonicotinsäure das

*5,10-Dihydro-5-[(1-methyl-1,2,5,6-tetrahydro-4-pyridinyl)carbonyl]-11H-dibenzo[b,e][1,4]diazepin-11-on-hemihydrat*

vom F. 233-23°C (Diisopropylether/Essigsäureethylester);

aus 5,10-Dihydro-11H-dibenzo[b,e][1,4]diazepin-11-on und Tropan-3α-essigsäure das

*endo-5,10-Dihydro-5-[(8-methyl-8-azabicyclo-[3,2,1]oct-3-yl)acetyl]-11H-dibenzo[b,e][1,4]diazepin-11-on*

vom F. 226-227°C (Essigsäureethylester);

aus 5,10-Dihydro-11H-dibenzo[b,e][1,4]diazepin-11-on und 1-Methyl-4-piperidinessigsäure das

*5,10-Dihydro-5-[(1-methyl-4-piperidinyl)acetyl]-11H-dibenzo[b,e][1,4]diazepin-11-on,*

nach Dünnschichtchromatogramm, OR- und $^1$H-NMR-Spektren identisch mit einem nach Beispiel 4 hergestellten Präparat;

aus 6-Chlor-5,10-dihydro-11H-dibenzo[b,e][1,4]-diazepin-11-on und 1-Methyl-4-piperidinessigsäure das

*6-Chlor-5,10-dihydro-5-[(1-methyl-4-piperidinyl)-acetyl]-11H-dibenzo[b,e][1,4]diazepin-11-on,*

nach Dünnschichtchromatogramm und IR- und NMR-Spektren identisch mit einem nach Beispiel 5 hergestellten Präparat.

*Beispiel 11*

*5,10-Dihydro-5-[(8-methyl-2,3-dehydro-8-aza-bicyclo[3,2,1]oct-3-yl)acetyl]-11H-dibenzo[b,e]-[1,4]diazepin-11-on* und *5,10-Dihydro-5-[(8-methyl-8-aza-bicyclo[3,2,1]-oct-3-yliden)acetyl]-11H-dibenzo[b,e][1,4]diazepin-11-on*

a) *5-Bromacetyl-5,10-dihydro-11H-dibenzo-[b,e][1,4]diazepin-11-on*

Zu einer Suspension von 105 g (0,5 Mol) 5,10-Dihydro-11H-dibenzo[b,e][1,4]diazepin-11-on in 1 *l* Toluol wurden unter Rühren bei 90°C 93 g (0,55 Mol) Bromacetylchlorid zugetropft. Anschliessend wurde weitere 30 Minuten unter Rückfluss erhitzt, wobei eine starke Chlorwasserstoff-Entwicklung auftrat. Nach dem Abkühlen wurde der erhaltene dunkelblau gefärbte Kristallbrei abgeasugt; F.: 215-216°C.;

Ausbeute: 143 g (86% der Theorie).

b) *5-Diethylphosphono-acetyl-5,10-dihydro-11H-dibenzo[b,e][1,4]diazepin-11-on*

Ein Gemisch aus 143 g (0,43 Mol) 5-Bromacetyl-5,10-dihydro-11H-dibenzo[b,e][1,4]diazepin-11-on und 400 ml Triethylphosphit wurde 2 Stunden unter Rühren bei 120°C unter Rückfluss erhitzt. Im Verlauf der Reaktion ging die 5-Bromacetyl-Verbindung völlig in Lösung und es begann die Abscheidung eines grauen Niederschlags. Nach dem Abkühlen wurde abgesaugt und mit Essigester nachgewaschen und es wurden 159 g (95% der Theorie) 5-Diethylphosphono-acetyl-5,10-dihydro-11H-dibenzo[b,e][1,4]diazepin-11-on erhalten; F.: 195 bis 197°C.

c) *5,10-Dihydro-5-[(8-methyl-2,3-dehydro-8-aza-bicyclo[3,2,1]oct-3-yl)acetyl]-11H-dibenzo-[b,e][1,4]diazepin-11-on* und *5,10-Dihydro-5-[(8-methyl-8-aza-bicyclo[3,2,1]-*

*oct-3-yliden)acetyl]-11H-dibenzo[b,e][1,4]diazepin-11-on*

In eine Suspension von 3,92 g (0,082 Mol) Natriumhydrid (50%ig in Paraffinöl) in 160 ml Tetrahydrofuran wurden unter Rühren bei Raumtemperatur portionsweise 15,9 g (0,041 Mol) 5-Diethylphosphono-acetyl-5,10-dihydro-11H-dibenzo[b,e][1,4]-diazepin-11-on eingetragen. Anschliessend wurde die Lösung eine Stunde unter Rückfluss erhitzt. Danach wurde eine Lösung von 5,7 g (0,041 Mol) Tropinon in 10 ml Tetrahydrofuran in das Reaktionsgemisch eingetropft und es wurde drei Tage unter leichtem Rückfluss erhitzt. Nach beendeter Reaktion wurde die Reaktionslösung mit 100 ml gesättigter Natriumchlorid-Lösung durchgeschüttelt. Die resultierende organische Phase wurde mit 100 ml Essigsäureethylester verdünnt und zweimal mit 10%iger Salzsäure ausgeschüttelt. Die salzsaure Phase wurde mit festem Kaliumcarbonat alkalisch gestellt und mit Essigsäureethylester extrahiert. Man saugte die Essigsäureethylesterlösung über Aktivkohle ab und engte nach dem Trocknen am Rotationsverdampfer ein. Als Rückstand resultierte ein braunes Öl, welches als Hauptkomponenten die beiden Produkte enthielt. Dies wurde säulenchromatographisch über Kieselgel (MN-Kieselgel 60; 0,063-0,2 mm Korngrösse; Elutionsmittel : Methylenchlorid/Methanol/Ammoniak = 90 : 9 : 1) aufgetrennt. Es wurden 5,1 g (33% der Theorie) 5,10-Dihydro-5-[(8-methyl-2,3-dehydro-8-aza-bicyclo[3,2,1]oct-3-yl)acetyl]-11H-dibenzo[b,e][1,4]diazepin-11-on als amorphes Pulver erhalten;

$C_{23}H_{23}N_3O_2$ (373,44)
Ber.: C 73,98 H 6,21 N 11,25
Gef.: C 74,12 H 6,18 N 11,20

Rf-Wert: 0,3 (Dünnschicht-Kieselgel-Chromatographie, Kieselgel 60 $F_{254}$, Schichtdicke 0,25 mm; Fliessmittel: Methylenchlorid/Methanol/Cyclohexan/Ammoniak = 68 : 15 : 15 : 2);

und 4,3 g (28% der Theorie) 5,10-Dihydro-5-[(8-methyl-8-aza-bicyclo[3,2,1]oct-3-yliden)acetyl]-11H-dibenzo[b,e][1,4]diazepin-11-on,

$C_{23}H_{23}N_3O_2$ (373,44)
Ber.: C 73,98 H 6,21 N 11,25
Gef.: C 73,90 H 6,25 N 11,21

Rf-Wert: 0,25 (Dünnschicht-Kieselgel-Chromatographie, Kieselgel 60 $F_{254}$, Schichtdicke 0,25 mm; Fliessmittel: Methylenchlorid/Methanol/Cyclohexan/Ammoniak = 68 : 15 : 15 : 2);

Analog werden erhalten:

*6-Chlor-5,10-dihydro-5-[(8-methyl-2,3-dehydro-8-aza-bicyclo[3,2,1]oct-3-yl)acetyl]-11H-dibenzo-[b,e][1,4]diazepin-11-on* und
*6-Chlor-5,10-dihydro-5-[(8-methyl-8-aza-bicyclo-[3,2,1]oct-3-yliden)acetyl]-11H-dibenzo[b,e][1,4]-diazepin-11-on*

aus 5-Bromoacetyl-6-chlor-5,10-dihydro-11H-dibenzo[b,e][1,4]diazepin-11-on über das

6-Chlor-5-diethylphosphono-acetyl-5,10-dihydro-11H-dibenzo[b,e][1,4]diazepin-11-on; und

*5,10-Dihydro-5-[(1-methyl-1,2,5,6-tetrahydro-4-pyridinyl)acetyl]-11H-dibenzo[b,e][1,4]diazepin-11-on*

aus 5-Diethylphosphono-acetyl-5,10-dihydro-11H-dibenzo[b,e][1,4]diazepin-11-on und N-Methyl-piperidin-4-on, und

*6-Chlor-5,10-dihydro-5-[(1-methyl-1,2,5,6-tetrahydro-4-pyridinyl)acetyl]-11H-dibenzo[b,e][1,4]diazepin-11-on*

aus 6-Chlor-5-diethylphosphono-acetyl-5,10-dihydro-11H-dibenzo[b,e][1,4]diazepin-11-on und N-Methyl-piperidin-4-on.

*Beispiel 12*

*5,10-Dihydro-5-[(1-methyl-4-piperidinyl)acetyl]-11H-dibenzo[b,e][1,4]diazepin-11-on*

3,5 g (0,01 Mol) 5,10-Dihydro-5-[(1-methyl-1,2,5,6-tetrahydro-4-pyridinyl)acetyl]-11H-dibenzo[b,e][1,4]diazepin-11-on wurden in 100 ml Ethanol gelöst und in Gegenwart von 0,5 g Platinoxid bei Raumtemperatur und unter einem Druck von 3 . $10^5$ Pa hydriert. Nach beendeter Wasserstoffaufnahme wurde vom Katalysator abfiltriert und im Vakuum eingedampft. Der Rückstand wurde säulenchromatographisch (NM-Kieselgel 60; 0,063-0,2 mm Korngrösse; Elutionsmittel: Essigester/Methanol = 9 : 1) gereinigt und ergab 3,2 g (91% der Theorie) 5,10-Dihydro-5-[(1-methyl-4-piperidinyl)acetyl]-11H-dibenzo[b,e][1,4]diazepin-11-on, das nach Dünnschichtchromatogramm, Elementaranalyse und IR-Spektrum identisch war mit dem nach Beispiel 4 erhaltenen Produkt.

Analog werden hergestellt:

*6-Chlor-5,10-dihydro-5-[(1-methyl-4-piperidinyl)-acetyl]-11H-dibenzo[b,e][1,4]diazepin-11-on*
aus
6-Chlor-5,10-dihydro-5-[(1-methyl-1,2,5,6-tetrahydro-4-pyridinyl)acetyl]-11H-dibenzo[b,e][1,4]diazepin-11-on;

*5,10-Dihydro-5-[(1-methyl-4-piperidinyl)acetyl]-11H-dibenzo[b,e][1,4]diazepin-11-on*
aus
5,10-Dihydro-5-[(1-methyl-4-pieridinyliden)acetyl]-11H-dibenzo[b,e][1,4]diazepin-11-on;

*6-Chlor-5,10-dihydro-5-[(1-methyl-4-piperidinyl)-acetyl]-11H-dibenzo[b,e][1,4]diazepin-11-on*
aus
6-Chlor-5,10-dihydro-5-[(1-methyl-4-piperidinyliden)acetyl]-11H-dibenzo[b,e][1,4]diazepin-11-on;

*endo-5,10-Dihydro-5-[(8-methyl-8-aza-bicyclo-[3,2,1]oct-3-yl)acetyl]-11H-dibenzo[b,e][1,4]diazepin-11-on* und
*exo-5,10-Dihydro-5-[(8-methyl-8-aza-bicyclo-[3,2,1]oct-3-yl)acetyl]-11H-dibenzo[b,e][1,4]diazepin-11-on*
aus
5,10-Dihydro-5-[(8-methyl-2,3-dehydro-8-aza-bicyclo[3,2,1]oct-3-yl)acetyl]-11H-dibenzo[b,e][1,4]-diazepin-11-on;

*endo-5,10-Dihydro-5-[(8-methyl-8-aza-bicyclo-[3,2,1]oct-3-yl)acetyl]-11H-dibenzo[b,e][1,4]diazepin-11-on* und *exo-5,10-Dihydro-5-[(8-methyl-8-aza-bicyclo-[3,2,1]oct-3-yl)acetyl]-11H-dibenzo[b,e][1,4]diazepin-11-on*
aus
5,10-Dihydro-5-[(8-methyl-8-aza-bicyclo[3,2,1]-oct-3-yliden)acetyl]-11H-dibenzo[b,e][1,4]diazepin-11-on;

*endo-6-Chlor-5,10-dihydro-5-[(8-methyl-8-aza-bicyclo[3,2,1]oct-3-yl)acetyl]-11H-dibenzo[b,e]-[1,4]diazepin-11-on* und *exo-6-Chlor-5,10-dihydro-5-[(8-methyl-8-aza-bicyclo[3,2,1]oct-3-yl)acetyl]-11H-dibenzo[b,e]-[1,4]diazepin-11-on*
aus
6-Chlor-5,10-dihydro-5-[(8-methyl-2,3-dehydro-8-aza-bicyclo[3,2,1]oct-3-yl)acetyl]-11H-dibenzo-[b,e][1,4]diazepin-11-on;

*endo-6-Chlor-5,10-dihydro-5-[(8-methyl-8-aza-bicyclo[3,2,1]oct-3-yl)acetyl]-11H-dibenzo[b,e]-[1,4]diazepin-11-on* und *exo-6-Chlor-5,10-dihydro-5-[(8-methyl-8-aza-bicyclo[3,2,1]oct-3-yl)acetyl]-11H-dibenzo[b,e]-[1,4]diazepin-11-on*
aus
6-Chlor-5,10-dihydro-5-[(8-methyl-8-aza-bicyclo-[3,2,1]oct-3-yliden)acetyl]-11H-dibenzo[b,e][1,4]-diazepin-11-on;

*5,10-Dihydro-5-[(1-methyl-4-piperidinyl)acetyl]-11H-dibenzo[b,e][1,4]diazepin-11-on*
aus
5,10-Dihydro-5-[(4-pyridinyl)acetyl]-11H-dibenzo-[b,e][1,4]diazepin-11-on-methojodid.

*Beispiel 13*

*6-Chlor-5,10-dihydro-5-[(1-methyl-4-piperidinyl)-acetyl]-11H-dibenzo[b,e][1,4]diazepin-11-on*

Zu einer Suspension von 5 g (0,02 Mol) 6-Chlor-5,10-]-11H-dibenzo[b,e][1,4]diazepin-11-on in 150 ml Tetrahydrofuran wurden 32 ml (0,05 Mol) n-Butyl-lithium (15%ig in n-Hexan), bei 0°C unter Rühren eingetropft. Danach wurde 30 Minuten bei Raumtemperatur gerührt. Anschliessend wurden 5,6 g (0,03 Mol) 1-Methylpiperidin-4-essigsäure-ethylester, in 25 ml Tetrahydrofuran gelöst, ebenfalls bei 0°C, unter Rühren, eingetropft und abschliessend 2 Stunden bei Raumtemperatur gerührt. Danach wurde das Reaktionsgemisch auf ca. 1/3 seines Volumens im Vakuum eingedampft und mit Eis/Wasser zersetzt. Nach dem Ansäuern mit verdünnter Salzsäure wurde dreimal mit Essigester ausgeschüttelt und die wässrige Phase in der Kälte mit festem Kaliumcarbonat alkalisch gestellt. Es wurde wieder mit Essigester ausgiebig extrahiert. Die Extrakte wurden getrocknet, filtriert und im Vakuum eingedampft. Nach Säulenchromatographie an Kieselgel wurden 1,7 g (22% der Theorie) 6-Chlor-5,10-dihydro-5-[(1-methyl-4-piperidinyl)acetyl]-11H-dibenzo[b,e][1,4]diazepin-11-on erhalten, das in seinen physikalischen Eigenschaften mit dem nach Beispiel 5 erhaltenen Produkt identisch war.

Im folgenden wird die Herstellung pharmazeutischer Anwendungsformen anhand einiger Beispiele beschrieben:

*Beispiel I*

*Tabletten mit 5 mg 6-Chlor-5,10-dihydro-5-[(1-methyl-4-piperidinyl)acetyl]-11H-dibenzo[b,e]-[1,4]diazepin-11-on*

Zusammensetzung:
1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

*Herstellungsverfahren:*

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpresst.

| | |
|---|---|
| Tablettengewicht: | 220 mg |
| Stempel | 9 mm |

*Beispiel II*

*Dragées mit 5 mg 6-Chlor-5,10-dihydro-5-[(1-methyl-4-piperidinyl)acetyl]-11H-dibenzo[b,e][1,4]diazepin-11-on*

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

*Beispiel III*

*Ampullen mit 1 mg 6-Chlor-5,10-dihydro-5-[(1-methyl-4-piperidinyl)acetyl]-11H-dibenzo[b,e][1,4]diazepin-11-on-hydrochlorid*

Zusammensetzung:
1 Ampulle enthält:

| | |
|---|---|
| Wirkstoff | 1,0 mg |
| Natriumchlorid | 8,0 mg |
| Dest. Wasser | ad 1 ml |

*Herstellungsverfahren:*

Die Wirksubstanz und Natriumchlorid werden in dest. Wasser gelöst und anschliessend auf das gegebene Volumen aufgefüllt. Die Lösung wird sterilfiltriert und in 1-ml-Ampullen abgefüllt. Sterilisation: 20 Minuten bei 120°C.

*Beispiel IV*

*Suppositorien mit 5 mg 6-Chlor-5,10-dihydro-5-[(1-methyl-4-piperidinyl)acetyl]-11H-dibenzo[b,e]-[1,4]diazepin-11-on*

Zusammensetzung:
1 Zäpfchen enthält:

| | |
|---|---|
| Wirkstoff | 5,0 mg |
| Zäpfchenmasse (z.B. Witepsol W 45 ®) | 1695,0 mg |
| | 1700,0 mg |

*Herstellungsverfahren:*

Die feinpulverisierte Wirksubstanz wird in der geschmolzenen und auf 40°C abgekühlten Zäpfchenmasse suspendiert. Man giesst die Masse bei 37°C in leicht vorgekühlte Zäpfchenformen aus.

Zäpfchengewicht: 1,7 g

*Beispiel V*

*Tropfen mit 6-Chlor-5,10-dihydro-5-[(1-methyl-4-piperidinyl)acetyl]-11H-dibenzo[b,e][1,4]diazepin-11-on*

Zusammensetzung:
100 ml Tropflösung enthalten:

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser ad | 100,0 ml |

*Herstellungsverfahren:*

Die Wirkstoffsubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wässrigen Lösung zu. Abschliessend wird mit Wasser auf 100 ml aufgefüllt und schwebeteilchenfrei filtriert.

**Patentansprüche für die Vertragsstaaten:**
BE, CH, DE, FR, IT, LI, LU, NL, SE

1. Substituierte Dibenzo-diazepinone der allgemeinen Formel I

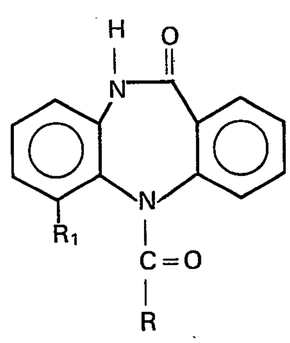

(I)

in der

$R_1$ ein Wasserstoff- oder Chloratom und

R eine, gegebenenfalls im heterocyclischen Ring durch eine oder zwei weitere Methylgruppen substituierte (1-Methyl-4-piperidinyl)-methyl-, (1-Methyl-1,2,5,6-tetrahydro-4-pyridinyl)-methyl-, 1-Methyl-1,2,5,6-tetrahydro-4-pyridinyl-, (1-Methyl-4-piperidinyliden)-methyl-, (2,3-Dehydro-8-methyl-8-aza-bicyclo[3,2,1]oct-3-yl)-methyl-, (8-Methyl-8-aza-bicyclo[3,2,1]oct-3-yliden)-methyl- oder eine endo- oder exo-(8-Methyl-8-aza-bicyclo[3,2,1]oct-3-yl)-methylgruppe bedeuten, ihre diastereomeren und enantiomeren Formen und ihre physiologisch verträglichen Säureadditionssalze mit anorganischen oder organischen Säuren.

2. Substituierte Dibenzodiazepine der allgemeinen Formel I gemäss Anspruch 1, dadurch gekennzeichnet, dass

$R_1$ ein Wasserstoff- oder Chloratom und

R die (1-Methyl-4-piperidinyl)-methyl-, 1-Methyl-1,2,5,6-tetrahydro-4-pyridinyl- oder die endo-(3-Methyl-8-aza-bicyclo[3,2,1]oct-3-yl)-methyl-Gruppe bedeuten und ihre physiologisch verträglichen Säureadditionssalze mit anorganischer oder organischen Säuren.

3. 6-Chlor-5,10-dihydro-5-[(1-methyl-4-piperidinyl)acetyl]-11H-dibenzo[b,e][1,4]diazepin-11-on und seine physiologisch verträglichen Salze.

4. Arzneimittel, enthaltend eine oder mehrere Verbindungen der allgemeinen Formel I gemäss Anspruch 1 neben den üblichen Träger- und/oder Hilfsstoffen.

5. Verfahren zur Herstellung neuer, substituierter Dibenzodiazepinone der allgemeinen Formel I

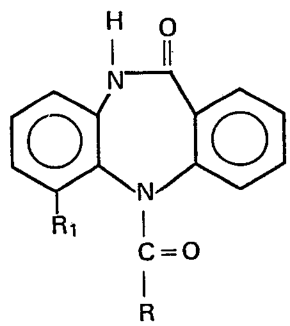

(I)

in der

$R_1$ ein Wasserstoff- oder Chloratom und

R eine, gegebenenfalls im heterocyclischen Ring durch eine oder zwei weitere Methylgruppen substituierten (1-Methyl-4-piperidinyl)-methyl-, (1-Methyl-1,2,5,6-tetrahydro-4-pyridinyl)-methyl-, 1-Methyl-1,2,5,6-tetrahydro-4-pyridinyl-, (1-Methyl-4-piperidinyliden)-methyl-, (2,3-Dehydro-8-methyl-8-aza-bicyclo[3,2,1]oct-3-yl)-methyl-, (8-Methyl-8-aza-bicyclo[3,2,1]oct-3-yliden)-methyl- oder eine endo- oder exo-(8-Methyl-8-aza-bicyclo[3,2,1]oct-3-yl)-methylgruppe bedeuten, und von deren Säureadditionssalzen mit anorganischen oder organischen Säuren, dadurch gekennzeichnet, dass

a) Dibenzodiazepinone der allgemeinen Formel II

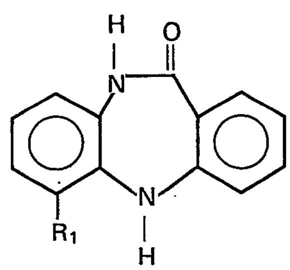

(II)

in der $R_1$ die oben angegebene Bedeutung hat, mit Säurederivaten der allgemeinen Formel III

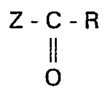

(III)

in der R wie oben definiert ist und Z eine nucleofuge Gruppe bedeutet, in einem inerten Lösungsmittel bei Temperaturen zwischen —25 und + 130°C acyliert werden oder

b) zur Herstellung von Verbindungen der allgemeinen Formel I, in der R eine gegebenenfalls im heterocyclischen Ring durch eine oder zwei weitere Methylgruppen substituierte (1-Methyl-1,2,5,6-tetrahydro-4-pyridinyl)-methyl oder 1-Methyl-1,2,-5,6-tetrahydro-4-pyridinylgruppe bedeutet, ein Pyridiniumsalz der allgemeinen Formel Va

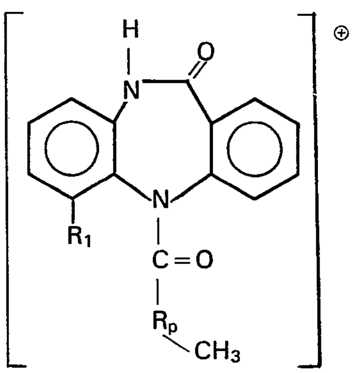

(Va)

in der $R_1$ wie oben definiert ist, X den Säurerest einer starken Sauerstoffsäure oder ein Halogenatom und $R_p$ eine gegebenenfalls durch eine oder zwei weitere Methylgruppen substituierte 4-Pyridinyl- oder (4--Pyridinyl)-methyl-Gruppe bedeuten, mit Borhydriden oder Alkoxyborhydriden in protischen Lösungsmitteln bei Temperaturen zwischen —40 und +50°C reduziert wird, oder

c) zur Herstellung von Verbindungen der allgemeinen Formel I, in der R einen im heterocyclischen Sechsring gegebenenfalls durch eine oder zwei weitere Methylgruppen substituierten (1-Methyl-1,2,-5,6-tetrahydro-4-pyridinyl)-methyl-, (1-Methyl-1,-2,5,6-tetrahydro-4-piperidinyliden)-methyl-, (2,3--Dehydro-8-methyl-8-aza-bicyclo[3,2,1]oct-3-yl)--methyl- oder (8-Methyl-8-aza-bicyclo[3,2,1]oct-3--yliden)-methyl-Rest bedeutet, ein 5-Dialkylphosphonoacetyldibenzodiazepinon der allgemeinen Formel VII

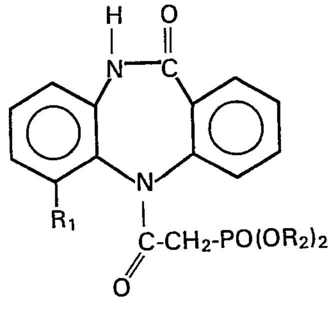

(VII)

in der $R_1$ wie oben definiert ist und $R_2$ eine Alkylgruppe mit 1 bis 10 Kohlenstoffatomen bedeutet, mit einem Piperidinon der allgemeinen Formel VIII oder einem Tropinon der allgemeinen Formel IX

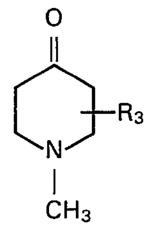

(VIII)

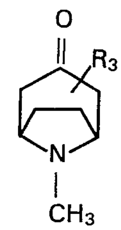

(IX)

in welchen $R_3$ ein Wasserstoffatom oder die Methylgruppe bedeutet, in Gegenwart eines Alkalihydrids oder Alkalialkoholats in einem Lösungsmittel bei Temperaturen von 20°C bis zum Siedepunkt des Reaktionsgemisches umgesetzt wird, oder

d) Zur Herstellung von Verbindungen der allgemeinen Formel I, in der R einen, im heterocyclischen Sechsring gegebenenfalls durch eine oder 2 weitere Methylgruppen substituierten (1-Methyl-4-piperidinyl)-methyl- oder einen endo- oder exo-(8-Methyl--8-aza-bicyclo[3,2,1]oct-3-yl)-methyl-Rest bedeutet, ein Dibenzodiazepinon der allgemeinen Formel X

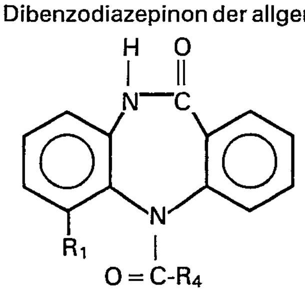

(X)

in der $R_1$ wie oben definiert ist und $R_4$ folgende Gruppen

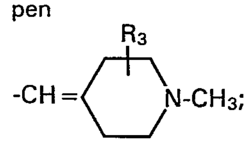

;

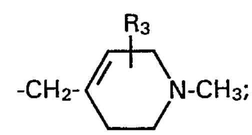

;

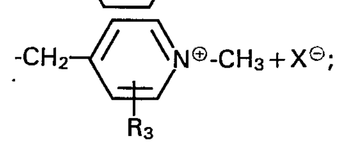

;

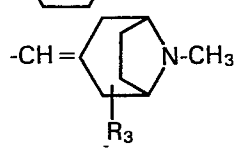

oder 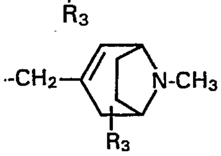 darstellt, wobei in diesen Gruppen $R_3$ ein Wasserstoffatom oder die Methylgruppe bedeutet und X ein Halogenatom ist, katalytisch hydriert wird oder

e) Dibenzodiazepinone der allgemeinen Formel II

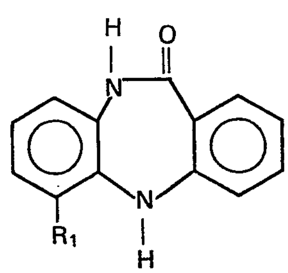

(II)

in der $R_1$ wie oben definiert ist, in einem organischen Lösungsmittel mit mindestens 2 Äquivalenten eines Lithiumalkyls oder Lithiumaryls oder Lithiumamid bei Temperaturen zwischen —60 und 0 °C in ihre Di-Lithiumsaze überführt werden und letztere anschliessend mit einem Ester der allgemeinen Formel XII

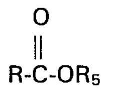
(XII)

in der R die eingangs erwähnten Bedeutungen besitzt und $R_5$ einen Alkylrest mit 1 bis 10 Kohlenstoffatomen oder einen Aralkylrest mit 7 bis 13 Kohlenstoffatomen darstellt, umgesetzt werden,
und gegebenenfalls eine so erhaltene Verbindung der allgemeinen Formel I anschliessend in ihre Salze mit anorganischen oder organischen Säuren übergeführt wird.

6. Verfahren gemäss Anspruch 5a, dadurch gekennzeichnet, dass als Säurederivat der allgemeinen Formel III ein Säurehalogenid, ein Ester, ein Säureanhydrid, ein gemischtes Säureanhydrid oder ein N--Alkyl-2-acyloxypyridiniumsalz verwendet und die Reaktion gegebenenfalls in einem inerten Lösungsmittel in Gegenwart eines säurebindenden Mittels durchgeführt wird.

7. Verfahren gemäss Anspruch 5b, dadurch gekennzeichnet, dass ein Pyridiniumsalz der allgemeinen Formel Va mittels Natrium- oder Kaliumtetrahydridoborat oder Natrium- oder Kaliumalkoxy-, dialkoxy- oder trialkoxyborhydriden bei —5 bis +10°C in Anwesenheit eines protischen Lösungsmittels reduziert wird.

8. Verfahren zur Herstellung der Verbindungen der allgemeinen Formel Va gemäss Anspruch 5b, dadurch gekennzeichnet, dass ein Dibenzodiazepinon der allgemeinen Formel II

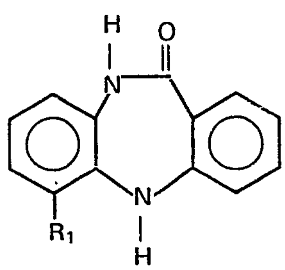
(II)

in der $R_1$ wie oben definiert ist, mit einem Acylierungsmittel der allgemeinen Formel IV

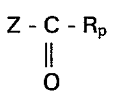
(IV)

in der Z eine nucleofuge Gruppe und $R_p$ eine gegebenenfalls durch eine oder zwei Methylgruppen substituierte 4-Pyridinyl- oder (4-Pyridinyl)-methyl-Gruppe darstellen, in einem inerten Lösungsmittel bei Temperaturen bis zum Siedepunkt des Reaktionsgemisches zu einer Verbindung der allgemeinen Formel V

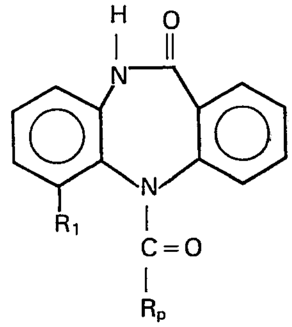
(V)

umgesetzt wird, und anschliessend eine so erhaltene Verbindung mit einem Methylierungsmittel der allgemeinen Formel VI

$$H_3C - X \quad (VI)$$

in der X den Säurerest einer starken Sauerstoffsäure oder ein Halogenatom bedeutet, in einem inerten Lösungsmittel bei Temperaturen zwischen —20 und +130°C zu der entsprechenden Verbindung der allgemeinen Formel Va methyliert wird.

9. Verfahren gemäss Anspruch 5d, dadurch gekennzeichnet, dass die Hydrierung mit Platindioxid oder Palladium auf Kohle oder Raney-Nickel oder Raney-Kobalt bei Temperaturen zwischen 10 und 40°C bei einem Wasserstoffdruck von $10^5$ bis $5 \cdot 10^6$ Pa durchgeführt wird.

10. Verwendung einer Verbindung der allgemeinen Formel I gemäss Anspruch 1 zur Herstellung von antiulcerogen wirkenden und magensäuresekretionshemmenden Arzneimitteln.

**Patentansprüche für den Vertragsstaat:** AT

1. Verfahren zur Herstellung substituierter Dibenzo-diazepinone der allgemeinen Formel I

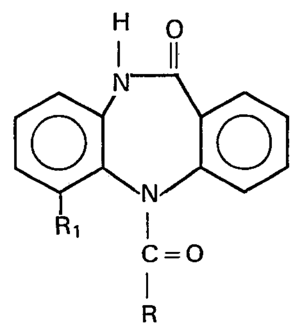
(I)

in der

$R_1$ ein Wasserstoff- oder Chloratom und

R eine, gegebenenfalls im heterocyclischen Ring durch eine oder zwei weitere Methylgruppen substituierte (1-Methyl-4-piperidinyl)-methyl-, (1-Methyl-1,2,5,6-tetrahydro-4-pyridinyl)-methyl-, 1-Methyl-1,2,5,6-tetrahydro-4-pyridinyl-, (1-Methyl-4--piperidinyliden)-methyl-, (2,3-Dehydro-8-methyl-8--aza-bicyclo[3,2,1]oct-3-yl)-methyl-, (8-Methyl-8--aza-bicyclo[3,2,1]oct-3-yliden)-methyl- oder eine endo- oder exo-(8-Methyl-8-aza-bicyclo[3,2,1]oct--3-yl)-methylgruppe bedeuten, und von deren Säureadditionssalzen mit anorganischen oder organischen Säuren, dadurch gekennzeichnet, dass

a) Dibenzodiazepinone der allgemeinen Formel II

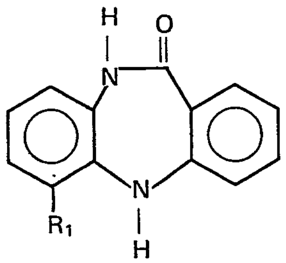

(II)

in der $R_1$ die oben angegebene Bedeutung hat, mit Säurederivaten der allgemeinen Formel III

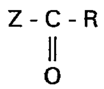

(III)

in der R wie oben definiert ist und Z eine nucleofuge Gruppe bedeutet, in einem inerten Lösungsmittel bei Temperaturen zwischen —25 und +130°C acyliert werden oder

b) zur Herstellung von Verbindungen der allgemeinen Formel I, in der R eine gegebenenfalls im heterocyclischen Ring durch eine oder zwei weitere Methylgruppen substituierte (1-Methyl-1,2,5,6-tetrahydro-4-pyridinyl)-methyl oder 1-Methyl-1,2,-5,6-tetrahydro-4-pyridinylgruppe bedeutet, ein Pyridiniumsalz der allgemeinen Formel Va

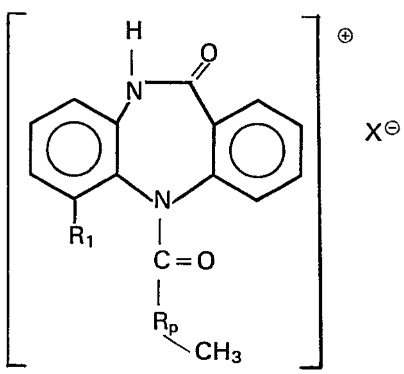

(Va)

in der $R_1$ wie oben definiert ist, X den Säurerest einer starken Sauerstoffsäure oder ein Halogenatom und $R_p$ eine gegebenenfalls durch eine oder zwei weitere Methylgruppen substituierte 4-Pyridinyl- oder (4--Pyridinyl)-methyl-Gruppe bedeuten, mit Borhydriden oder Alkoxyborhydriden in protischen Lösungsmitteln bei Temperaturen zwischen —40 und +50°C reduziert wird, oder

c) zur Herstellung von Verbindungen der allgemeinen Formel I, in der R einen im heterocyclischen Sechsring gegebenenfalls durch eine oder zwei weitere Methylgruppen substituierten (1-Methyl-1,2,-5,6-tetrahydro-4-pyridinyl)-methyl-, (1-Methyl-1,-2,5,6-tetrahydro-4-piperidinyliden)-methyl-, (2,3--Dehydro-8-methyl-8-aza-bicyclo[3,2,1]oct-3-yl)--methyl- oder (8-Methyl-8-aza-bicyclo[3,2,1]oct-3--yliden)-methyl-Rest bedeutet, ein 5-Dialkylphosphonoacetyldibenzodiazepinon der allgemeinen Formel VII

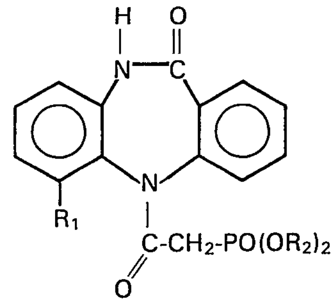

(VII)

in der $R_1$ wie oben definiert ist und $R_2$ eine Alkylgruppe mit 1 bis 10 Kohlenstoffatomen bedeutet, mit einem Piperidinon der allgemeinen Formel VIII oder einem Tropinon der allgemeinen Formel IX

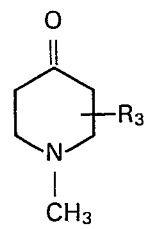

(VIII)

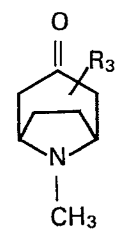

(IX)

in welchen $R_3$ ein Wasserstoffatom oder die Methylgruppe bedeutet, in Gegenwart eines Alkalihydrids oder Alkalialkoholats in einem Lösungsmittel bei Temperaturen von 20°C bis zum Siedepunkt des Reaktionsgemisches umgesetzt wird, oder

d) zur Herstellung von Verbindungen der allgemeinen Formel I, in der R einen, im heterocyclischen Sechsring gegebenenfalls durch eine oder 2 weitere Methylgruppen substituierten (1-Methyl-4-piperidinyl)-methyl- oder einen endo- oder exo-(8-Methyl--8-aza-bicyclo[3,2,1]oct-3-yl)-methyl-Rest bedeutet, ein Dibenzodiazepinon der allgemeinen Formel X

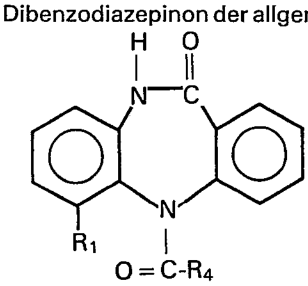

(X)

in der $R_1$ wie oben definiert ist und $R_4$ folgende Gruppen

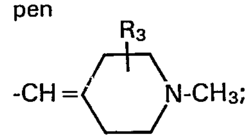; 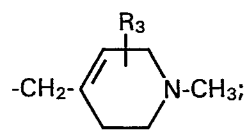;

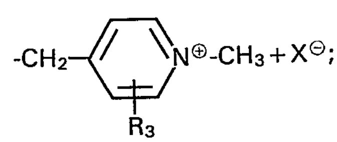; 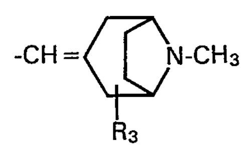

oder 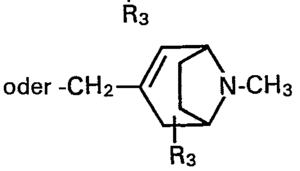 darstellt, wobei in diesen Gruppen $R_3$ ein Wasserstoffatom oder die Methylgruppe bedeutet und X ein Halogenatom ist, katalytisch hydriert wird oder

e) Dibenzodiazepinone der allgemeinen Formel II

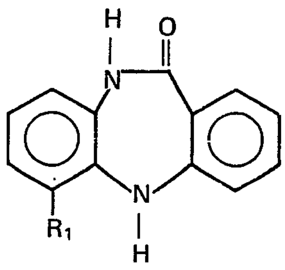

(II)

in der $R_1$ wie oben definiert ist, in einem organischen Lösungsmittel mit mindestens 2 Äquivalenten eines Lithiumalkyls oder Lithiumaryls oder Lithiumamid bei Temperaturen zwischen —60 und 0°C in ihre Di-Lithiumsalze überführt werden und letztere anschliessend mit einem Ester der allgemeinen Formel XII

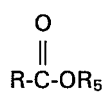

(XII)

in der R die eingangs erwähnten Bedeutungen besitzt und $R_5$ einen Alkylrest mit 1 bis 10 Kohlenstoffatomen oder einen Aralkylrest mit 7 bis 13 Kohlenstoffatomen darstellt, umgesetzt werden,
und gegebenenfalls eine so erhaltene Verbindung der allgemeinen Formel I anschliessend in ihre Salze mit anorganischen oder organischen Säuren übergeführt wird.

2. Verfahren zur Herstellung substituierter Dibenzo-diazepinone der allgemeinen Formel I

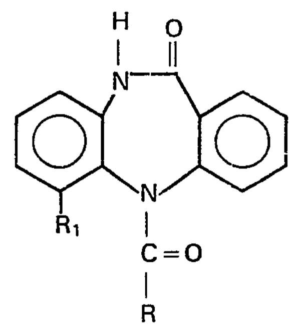

(I)

in der
$R_1$ ein Wasserstoff- oder Chloratom und
R eine, gegebenenfalls im heterocyclischen Ring durch eine oder zwei weitere Methylgruppen substituierte (1-Methyl-4-piperidinyl)-methyl-, (1-Methyl-1,2,5,6-tetrahydro-4-pyridinyl)-methyl-, 1-Methyl-1,2,5,6-tetrahydro-4-pyridinyl-, (1-Methyl-4-piperidinyliden)-methyl-, oder eine endo- oder exo-(8-Methyl-8-aza-bicyclo[3,2,1]oct-3-yl)-methylgruppe bedeuten, und von deren Säureadditionssalzen mit anorganischen oder organischen Säuren, dadurch gekennzeichnet, dass

a) Dibenzodiazepinone der allgemeinen Formel II

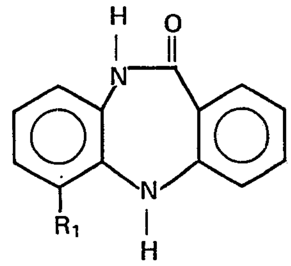

(II)

in der $R_1$ die oben angegebene Bedeutung hat, mit Säurederivaten der allgemeinen Formel III

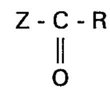

(III)

in der R wie oben definiert ist und Z eine nucleofuge Gruppe bedeutet, in einem inerten Lösungsmittel bei Temperaturen zwischen —25 und +130°C acyliert werden oder

b) zur Herstellung von Verbindungen der allgemeinen Formel I, in der R eine gegebenenfalls im heterocyclischen Ring durch eine oder zwei weitere Methylgruppen substituierte (1-Methyl-1,2,5,6-tetrahydro-4-pyridinyl)-methyl oder 1-Methyl-1,2,-5,6-tetrahydro-4-pyridinylgruppe bedeutet, ein Pyridiniumsalz der allgemeinen Formel
in der $R_1$ wie oben definiert ist, X den Säurerest einer starken Sauerstoffsäure oder ein Halogenatom und $R_p$ eine gegebenenfalls durch eine oder zwei weitere Methylgruppen substituierte 4-Pyridinyl- oder (4-Pyridinyl)-methyl-Gruppe bedeuten,

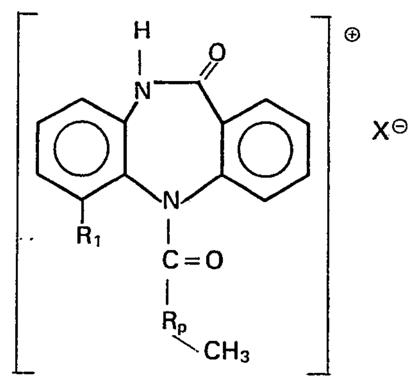

(Va)

mit Borhydriden oder Alkoxyborhydriden in protischen Lösungsmitteln bei Temperaturen zwischen —40 und +50°C reduziert wird,
und gegebenenfalls eine so erhaltene Verbindung der allgemeinen Formel I anschliessend in ihre Salze mit anorganischen oder organischen Säuren übergeführt wird.

3. Verfahren gemäss Anspruch 1a, dadurch gekennzeichnet, dass als Säurederivat der allgemeinen Formel III ein Säurehalogenid, ein Ester, ein Säureanhydrid, ein gemischtes Säureanhydrid oder ein N-Alkyl-2-acyloxypyridiniumsalz verwendet und die Reaktion gegebenenfalls in einem inerten Lösungsmittel in Gegenwart eines säurebindenden Mittels durchgeführt wird.

4. Verfahren gemäss Anspruch 1b, dadurch gekennzeichnet, dass ein Pyridiniumsalz der allgemeinen Formel Va mittels Natrium- oder Kaliumtetrahydridoborat oder Natrium- oder Kaliumalkoxy-, dialkoxy- oder trialkoxyborhydriden bei —5 bis +10°C in Anwesenheit eines protischen Lösungsmittels reduziert wird.

5. Verfahren zur Herstellung der Verbindungen der allgemeinen Formel Va gemäss Anspruch 1b, dadurch gekennzeichnet, dass ein Dibenzodiazepinon der allgemeinen Formel II

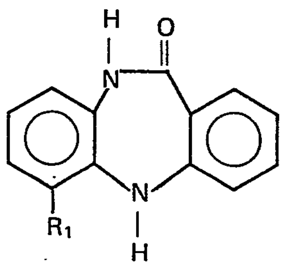

(II)

in der $R_1$ wie oben definiert ist, mit einem Acylierungsmittel der allgemeinen Formel IV

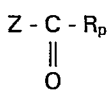

(IV)

in der Z eine nucleofuge Gruppe und $R_p$ eine gegebenenfalls durch eine oder zwei Methylgruppen substituierte 4-Pyridinyl- oder (4-Pyridinyl)-methyl-Gruppe darstellen, in einem inerten Lösungsmittel bei Temperaturen bis zum Siedepunkt des Reaktionsgemisches zu einer Verbindung der allgemeinen Formel V

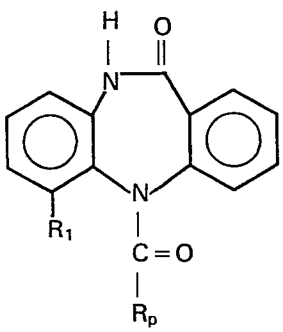

(V)

umgesetzt wird, und anschliessend eine so erhaltene Verbindung mit einem Methylierungsmittel der allgemeinen Formel VI

$$H_3C - X \quad \text{(VI)}$$

in der X den Säurerest einer starken Sauerstoffsäure oder ein Halogenatom bedeutet, in einem inerten Lösungsmittel bei Temperaturen zwischen —20 und +130°C zu der entsprechenden Verbindung der allgemeinen Formel Va methyliert wird.

6. Verfahren gemäss Anspruch 1d, dadurch gekennzeichnet, dass die Hydrierung mit Platindioxid oder Palladium auf Kohle oder Raney-Nickel oder Raney-Kobalt bei Temperaturen zwischen 10 und 40°C bei einem Wasserstoffdruck von $10^5$ bis 5 . $10^6$ Pa durchgeführt wird.

**Claims for the contracting states:**
BE, CH, DE, FR, IT, LI, LU, NL, SE

1. Substituted dibenzodiazepinones of general formula I

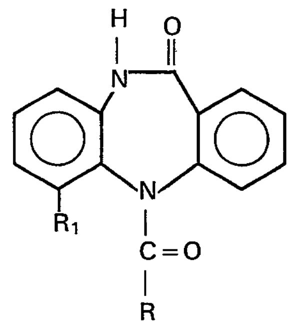

(I)

wherein
$R_1$ represents a hydrogen or chlorine atom and
R represents a (1-methyl-4-piperidinyl)methyl, (1-methyl-1,2,5,6-tetrahydro-4-pyridinyl)methyl, 1-methyl-1,2,5,6-tetrahydro-4-pyridinyl, (1-methyl-4-piperidinylidene)methyl, (2,3-dehydro-8-methyl-8-aza-bicyclo[3,2,1]oct-3-yl)-methyl, (8-methyl-8-aza-bicyclo[3,2,1]oct-3-ylidene)-methyl or an endo- or exo-(8-methyl-8-azabicyclo[3,2,1]oct--3-yl)methyl group, each group being optionally substituted by one or two further methyl groups in the heterocyclic ring, the diastereomeric and enantiomeric forms thereof and the physiologically acceptable acid addition salts thereof with inorganic or organic acids.

2. Substituted dibenzodiazepinones of general formula I as claimed in claim 1, characterised in that
$R_1$ represents a hydrogen or chlorine atom and
R represents the (1-methyl-4-piperidinyl)methyl, 1-methyl-1,2,5,6-tetrahydro-4-pyridinyl or endo-(8--methyl-8-aza-bicyclo[3,2,1]oct-3-yl)-methyl group, and the physiologically acceptable acid addition salts thereof with inorganic or organic acids.

3. 6-Chloro-5,10-dihydro-5-[(1-methyl-4-piperidinyl)-acetyl]-11H-dibenzo[b,e][1,4]diazepin-11--one and the physiologically acceptable salts thereof.

4. Pharmaceutical compositions containing one or more compounds of general formula I as claimed in claim 1 together with the conventional carriers and/or excipients.

5. Process for preparing new substituted dibenzodiazepinones of general formula I

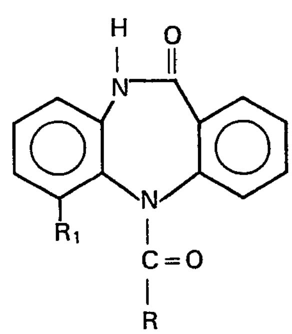

(I)

wherein
$R_1$ represents a hydrogen or chlorine atom and

R represents a (1-methyl-4-piperidinyl)methyl, (1-methyl-1,2,5,6-tetrahydro-4-pyridinyl)methyl, 1-methyl-1,2,5,6-tetrahydro-4-pyridinyl, (1-methyl-4-piperidinylidene)methyl, (2,3-dehydro-8-methyl-8-aza-bicyclo[3,2,1]oct-3-yl)-methyl, (8-methyl-8-aza-bicyclo[3,2,1]oct-3-ylidene)-methyl or an endo- or exo-(8-methyl-8-azabicyclo[3,2,1]oct-3-yl)methyl group, each group being optionally substituted by one or two further methyl groups in the heterocyclic ring, and of the acid addition salts thereof with inorganic or organic acids, characterised in that

a) dibenzodiazepinones of general formula II

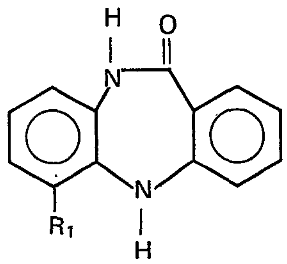

(II)

wherein $R_1$ is as hereinbefore defined, are acylated with acid derivatives of general formula III

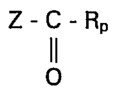

(III)

wherein R is as hereinbefore defined and Z represents a nucleophobic group, in an inert solvent at temperatures of between —25 and +130°C, or

b) in order to prepare compounds of general formula I wherein R represents a (1-methyl-1,2,5,6-tetrahydro-4-pyridinyl)methyl or 1-methyl-1,2,-5,6-tetrahydro-4-pyridinyl group optionally substituted by one or two further methyl groups in the heterocyclic ring, a pyridinium salt of general formula Va

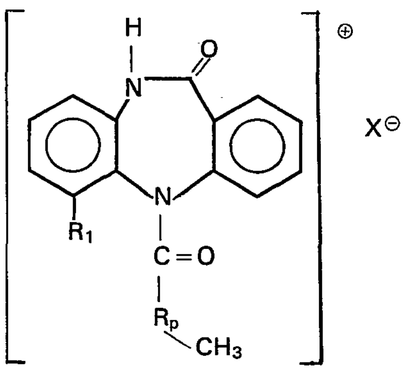

(Va)

wherein $R_1$ is as hereinbefore defined, X represents the acid group of a strong oxyacid or a halogen atom and $R_p$ represents a 4-pyridinyl or (4-pyridinyl)methyl group optionally substituted by one or two further methyl groups, is reduced with borohydrides or alkoxyborohydrides in protic solvents at temperatures of between —40 and +50°C, or

c) in order to prepare compounds of general formula I wherein R represents a (1-methyl-1,2,5,6-tetrahydro-4-pyridinyl)methyl, (1-methyl-1,2,5,6-tetrahydro-4-piperidinylidene)methyl, (2,3-dehydro-8-methyl-8-aza-bicyclo[3,2,1]oct-3-yl)methyl or (8-methyl-8-aza-bicyclo[3,2,1]oct-3-ylidene)methyl group, each group being optionally substituted by one or two further methyl groups in the heterocyclic six-membered ring, a 5-dialkylphosphonoacetyldibenzodiazepinone of general formula VII

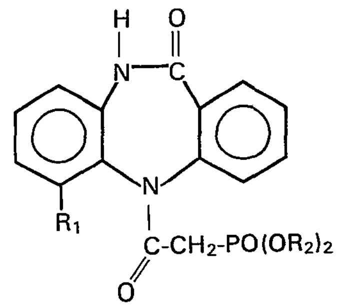

(VII)

wherein $R_1$ is as hereinbefore defined and $R_2$ represents an alkyl group with 1 to 10 carbon atoms, is reacted with a piperidinone of general formula VIII or a tropinone of general formula IX

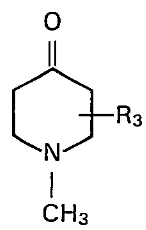

(VIII)

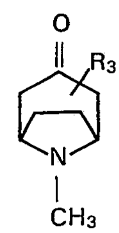

(IX)

wherein $R_3$ represents a hydrogen atom or the methyl group, in the presence of an alkali metal hydride or an alkali metal alkoxide in a solvent at temperatures of from 20°C up to the boiling point of the reaction mixture, or

d) in order to prepare compounds of general formula I wherein R represents a (1-methyl-4-piperidinyl)methyl or an endo- or exo-(8-methyl-8-aza-bicyclo[3,2,1]oct-3-yl)-methyl group, optionally substituted by one or two further methyl groups in the heterocyclic six-membered ring, a dibenzodiazepinone of general formula X

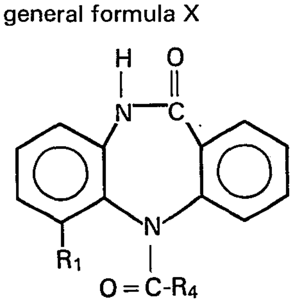

(X)

wherein $R_1$ is as hereinbefore defined and $R_4$ represents the following groups

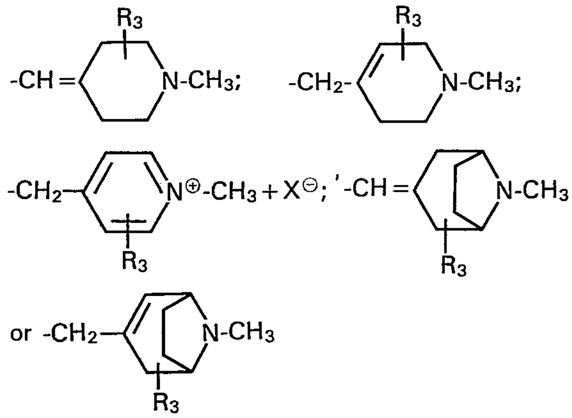

or

wherein $R_3$ represents a hydrogen atom or the methyl group and X is a halogen atom, is catalytically hydrogenated or

e) dibenzodiazepinones of general formula II

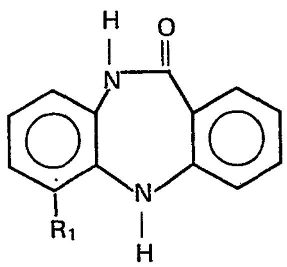

(II)

wherein $R_1$ is as hereinbefore defined, are converted into the di-lithium salts thereof in an organic solvent with at least two equivalents of a lithium alkyl or lithium aryl or lithium amide at temperatures of between —60 and 0°C, and these di-lithium salts are subsequently reacted with an ester of general formula XII

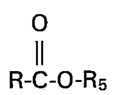

(XII)

wherein R is as hereinbefore defined and $R_5$ represents an alkyl group with 1 to 10 carbon atoms or an aralkyl group with 7 to 13 carbon atoms,
and if required a compound of general formula I thus obtained is subsequently converted into the salts thereof with inorganic or organic acids.

6. Process as claimed in claim 5a, characterised in that the acid derivative of general formula III used is an acid halide, an erster, an acid anhydride, a mixed acid anhydride or an N-alkyl-2-acyloxypyridinium salt, and the reaction is optionally carried out in an inert solvent in the presence of an acid-binding agent.

7. Process as claimed in claim 5b, characterised in that a pyridinium salt of general formula Va is reduced by means of sodium or potassium tetrahydridoborate or sodium or potassium alkoxy-, dialkoxy- or trialkoxy-borohydrides at —5 to +10°C in the presence of a protic solvent.

8. Process for preparing compounds of general formula Va as claimed in claim 5b, characterised in that a dibenzodiazepinone of general formula II

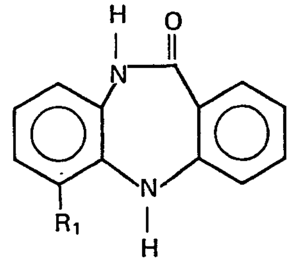

(II)

wherein $R_1$ is as hereinbefore defined, is reacted with an acylating agent of general formula IV

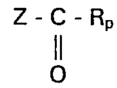

(IV)

wherein Z represents a nucleophobic group and $R_p$ represents a 4-pyridinyl or (4-pyridinyl)methyl group optionally substituted by one or two methyl groups, in an inert solvent at temperatures up to the boiling point of the reaction mixture to form a compound of general formula V

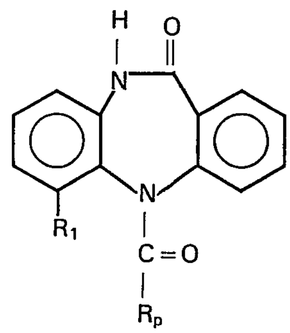

(V)

and subsequently a compound thus obtained is methylated with a methylating agent of general formula

$$H_3C - X \qquad (VI)$$

wherein X represents the acid group of a strong oxyacid or a halogen atom, in an inert solvent at temperatures of between —20 and +130°C to form the corresponding compound of general formula Va.

9. Process as claimed in claim 5d, characterised in that the hydrogenation is carried out with platinum dioxide or palladium on charcoal or Raney nickel or Raney cobalt at temperatures of between 10 and 40°C at a hydrogen pressure of from $10^5$ to $5 . 10^6$ Pa.

10. Use of a compound of general formula I as claimed in claim 1 for the preparation of pharmaceutical compositions with an antiulcerative effect and an inhibitory effect on the secretion of gastric acid.

**Claims for the contracting state:** AT

1. Process for preparing substituted dibenzodiazepinones of general formula I

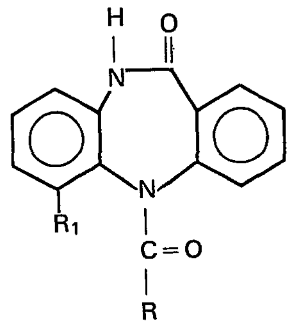

(I)

wherein

$R_1$ represents a hydrogen or chlorine atom and

R represents a (1-methyl-4-piperidinyl)methyl, (1-methyl-1,2,5,6-tetrahydro-4-pyridinyl)methyl, 1-methyl-1,2,5,6-tetrahydro-4-pyridinyl, (1-methyl-4-piperidinylidene)methyl, (2,3-dehydro-8-methyl-8-aza-bicyclo[3,2,1]oct-3-yl)-methyl, (8-methyl-8-aza-bicyclo[3,2,1]oct-3-ylidene)-methyl or an <u>endo</u>- or <u>exo</u>-(8-methyl-8-azabicyclo[3,2,1]oct-3-yl)methyl group, each group being optionally substituted by one or two further methyl groups in the heterocyclic ring, and of the acid addition salts thereof with inorganic or organic acids, characterised in that

a) dibenzodiazepinones of general formula II

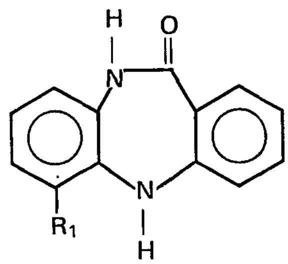

(II)

wherein $R_1$ is as hereinbefore defined, are acylated with acid derivatives of general formula III

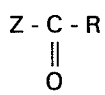

(III)

wherein R is as hereinbefore defined and Z represents a nucleophobic group, in an inert solvent at temperatures of between —25 and +130°C, or

b) in order to prepare compounds of general formula I wherein R represents a (1-methyl-1,2,5,6-tetrahydro-4-pyridinyl)methyl or 1-methyl-1,2,5,6-tetrahydro-4-pyridinyl group optionally substituted by one or two further methyl groups in the heterocyclic ring, a pyridinium salt of general formula Va

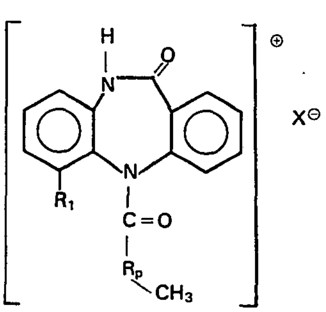

(Va)

wherein $R_1$ is as hereinbefore defined, X represents the acid group of a strong oxyacid or a halogen atom and $R_p$ represents a 4-pyridinyl or (4-pyridinyl)methyl group optionally substituted by one or two further methyl groups, is reduced with borohydrides or alkoxyborohydrides in protic solvents at temperatures of between —40 and +50°C, or

c) in order to prepare compounds of general formula I wherein R represents a (1-methyl-1,2,5,6-tetrahydro-4-pyridinyl)methyl, (1-methyl-1,2,5,6-tetrahydro-4-piperidinylidene)methyl, (2,3-dehydro-8-methyl-8-aza-bicyclo[3,2,1]oct-3-yl)methyl or (8-methyl-8-aza-bicyclo[3,2,1]oct-3-ylidene)methyl group, each group being optionally substituted by one or two further methyl groups in the heterocyclic six-membered ring, a 5-dialkylphosphonoacetyldibenzodiazepinone of general formula VII

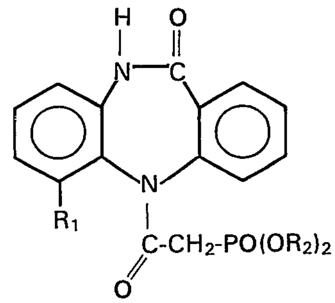

(VII)

wherein $R_1$ is as hereinbefore defined and $R_2$ represents an alkyl group with 1 to 10 carbon atoms, is reacted with a piperidinone of general formula VIII or a tropinone of general formula IX

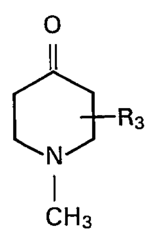

(VIII)

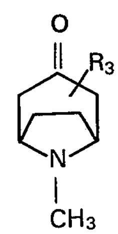

(IX)

wherein $R_3$ represents a hydrogen atom or the methyl group, in the presence of an alkali metal hydride or an alkali metal alkoxide in a solvent at temperatures of from 20°C up to the boiling point of the reaction mixture, or

d) in order to prepare compounds of general formula I wherein R represents a (1-methyl-4-piperidinyl)methyl or an <u>endo</u>- or <u>exo</u>-(8-methyl-8-aza-bicyclo[3,2,1]oct-3-yl)-methyl group, optionally substituted by one or two further methyl groups in the heterocyclic six-membered ring, a dibenzodiazepinone of general formula X

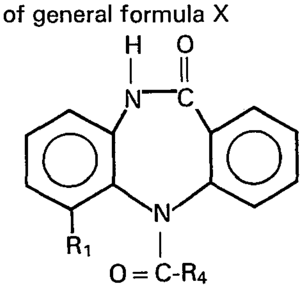

(X)

wherein $R_1$ is as hereinbefore defined and $R_4$ represents the following groups

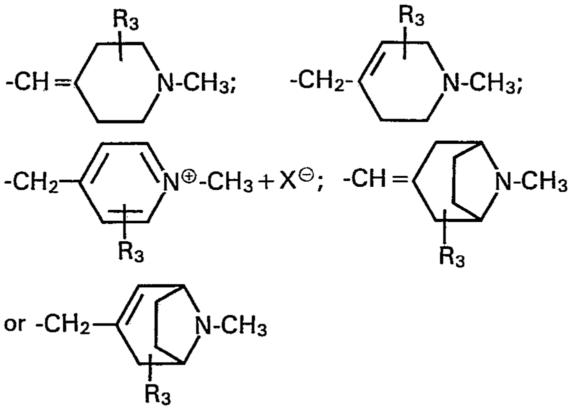

wherein $R_3$ represents a hydrogen atom or the methyl group and X is a halogen atom, is catalytically hydrogenated or

e) dibenzodiazepinones of general formula II

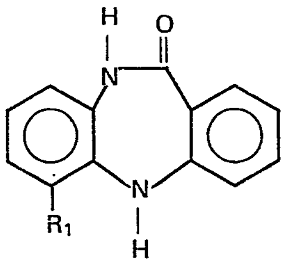

(II)

wherein $R_1$ is as hereinbefore defined, are converted into the di-lithium salts thereof in an organic solvent with at least two equivalents of a lithium alkyl or lithium aryl or lithium amide at temperatures of between —60 and 0°C, and these di-lithium salts are subsequently reacted with an ester of general formula XII

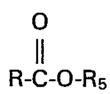

(XII)

wherein R is as hereinbefore defined and $R_5$ represents an alkyl group with 1 to 10 carbon atoms or an aralkyl group with 7 to 13 carbon atoms,
and if required a compound of general formula I thus obtained is subsequently converted into the salts thereof with inorganic or organic acids.

2. Process for preparing substituted dibenzodiazepinones of general formula I

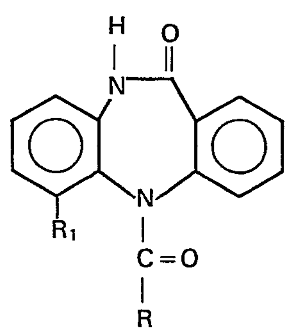

(I)

wherein

$R_1$ represents a hydrogen or chlorine atom and

R represents a (1-methyl-4-piperidinyl)methyl, (1-methyl-1,2,5,6-tetrahydro-4-pyridinyl)methyl, 1-methyl-1,2,5,6-tetrahydro-4-pyridinyl, (1-methyl-4-piperidinylidene)methyl or an endo- or exo-(8-methyl-8-azabicyclo[3,2,1]oct-3-yl)methyl group, each group being optionally substituted by one or two further methyl groups in the heterocyclic ring, and of the acid addition salts thereof with inorganic or organic acids, characterised in that

a) dibenzodiazepinones of general formula II

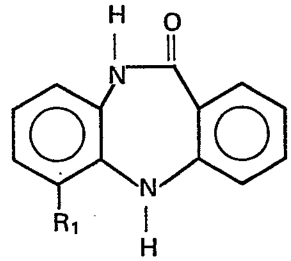

(II)

wherein $R_1$ is as hereinbefore defined, are acylated with acid derivatives of general formula III

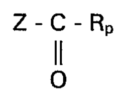

(III)

wherein R is as hereinbefore defined and Z represents a nucleophobic group, in an inert solvent at temperatures of between —25 and +130°C, or

b) in order to prepare compounds of general formula I wherein R represents a (1-methyl-1,2,5,6-tetrahydro-4-pyridinyl)methyl or 1-methyl-1,2,5,6-tetrahydro-4-pyridinyl group optionally substituted by one or two further methyl groups in the heterocyclic ring, a pyridinium salt of general formula Va

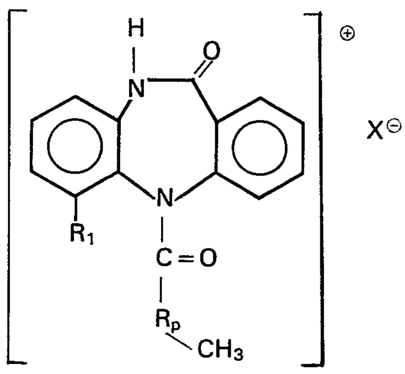

(Va)

wherein $R_1$ is as hereinbefore defined, X represents the acid group of a strong oxyacid or a halogen atom and $R_p$ represents a 4-pyridinyl or (4-pyridinyl)methyl group optionally substituted by one or two further methyl groups, is reduced with borohydrides or alkoxyborohydrides in protic solvents at temperatures of between —40 and +50°C,
and if required a compound of general formula I thus obtained is subsequently converted into the salts thereof with inorganic or organic acids.

3. Process as claimed in claim 1a, characterised in that the acid derivative of general formula III used is an acid halide, an ester, an acid anhydride, a mixed acid anhydride or an N-alkyl-2-acyloxypyridinium salt, and the reaction is optionally carried out in an inert solvent in the presence of an acid-binding agent.

4. Process as claimed in claim 1b, characterised in that a pyridinium salt of general formula Va is reduced by means of sodium or potassium tetrahydridoborate or sodium or potassium alkoxy-, dialkoxy- or trialkoxy-borohydrides at —5 to +10°C in the presence of a protic solvent.

5. Process for preparing compounds of general formula Va as claimed in claim 1b, characterised in that a dibenzodiazepinone of general formula II

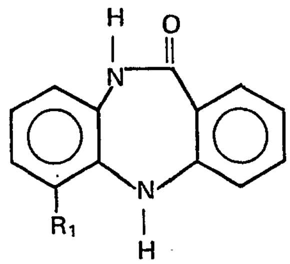

(II)

wherein $R_1$ is as hereinbefore defined, is reacted with an acylating agent of general formula IV

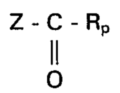

(IV)

wherein Z represents a nucleophobic group and $R_p$ represents a 4-pyridinyl or (4-pyridinyl)methyl group optionally substituted by one or two methyl groups, in an inert solvent at temperatures up to the boiling point of the reaction mixture to form a compound of general formula V

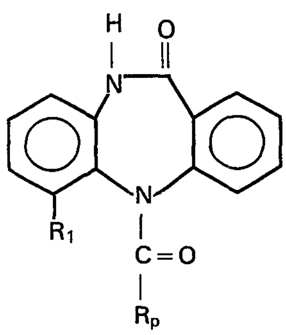

(V)

and subsequently a compound thus obtained is methylated with a methylating agent of general formula VI

$$H_3C - X \qquad (VI)$$

wherein X represents the acid group of a strong oxyacid or a halogen atom, in an inert solvent at temperatures of between —20 and +130°C to form the corresponding compound of general formula Va.

6. Process as claimed in claim 1d, characterised in that the hydrogenation is carried out with platinum dioxide or palladium on charcoal or Raney nickel or Raney cobalt at temperatures of between 10 and 40°C at a hydrogen pressure of from $10^5$ to $5.10^6$ Pa.

**Revendications pour les états contractants:**
BE, CH, DE, FR, IT, LI, LU, NL, SE

1. Dibenzodiazépinones substituées de formule générale I

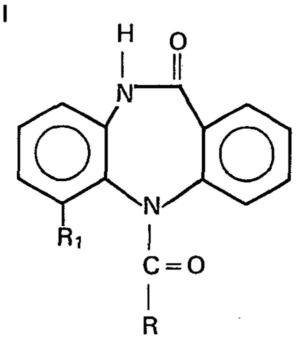

(I)

dans laquelle

$R_1$ représente un atome d'hydrogène ou de chlore et

R représente un groupe (1-méthyl-4-pipéridinyl)--méthyle, (1-méthyl-1,2,5,6-tétrahydro-4-pyridinyl)-méthyle, 1-méthyl-1,2,5,6-tétrahydro-4-pyridinyle, (1-méthyl-4-pipéridinylidène)-méthyle, (2,3--déhydro-8-méthyl-8-aza-bicyclo[3,2,1]oct-3-yl)--méthyle, (8-méthyle-8-aza-bicyclo[3,2,1]oct-3--ylidène)-méthyle éventuellement substitué dans le cycle hétérocyclique par un ou deux autres groupes méthyle, or représente un groupe endo- ou exo-(8--méthyl-8-azabicyclo[3,2,1]oct-3-yl)-méthyle, leurs formes diastéréoisomères et énantiomères et leurs sels d'addition d'acides physiologiquement supportables avec des acides minéraux ou organiques.

2. Dibenzodiazépinones substituées de formule générale I selon la revendication 1, caractérisées en ce que

$R_1$ représente un atome d'hydrogène ou de chlore et

R représente le groupe (1-méthyl-4-pipéridinyl)--méthyle, 1-méthyl-1,2,5,6-tétrahydro-4-pyridinyle ou le groupe endo-(3-méthyl-8-aza-bicyclo[3,2,1]--oct-3-yl)-méthyle et leurs sels d'addition d'acides physiologiquement supportables avec des acides minéraux ou organiques.

3. La 6-chloro-5,10-dihydro-5-[(1-méthyl-4-pipéridinyl)acétyl]-11H-dibenzo[b,e][1,4]diazépine--11-one et ses sels physiologiquement supportables.

4. Médicament contenant un ou plusieurs composés de formule générale I selon la revendication 1 conjointement aux excipients et/ou adjuvants usuels.

5. Procédé pour la préparation de nouvelles dibenzodiazépinones substituées de formule générale I

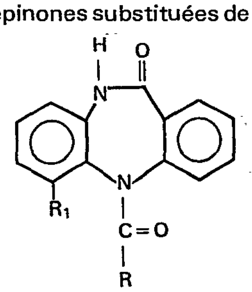

(I)

dans laquelle

$R_1$ représente un atome d'hydrogène ou de chlore et

R représente un groupe (1-méthyl-4-pipéridinyl)-méthyle, (1-méthyl-1,2,5,6-tétrahydro-4-pyridinyl)-méthyle, 1-méthyl-1,2,5,6-tétrahydro-4-pyridinyle, (1-méthyl-4-pipéridinylidène)-méthyle, (2,3-déhydro-8-méthyl-8-aza-bicyclo[3,2,1]oct-3-yl)-méthyle, (8-méthyle-8-aza-bicyclo[3,2,1]oct-3-ylidène)-méthyle éventuellement substitué dans le cycle hétérocyclique par un ou deux autres groupes méthyle, ou représente un groupe endo- ou exo-(8-méthyl-8-azabicyclo[3,2,1]oct-3-yl)-méthyle, et de leurs sels d'addition d'acides avec des acides minéraux ou organiques, caractérisé en ce que:

a) on acyle des dibenzodiazépinones de formule générale II

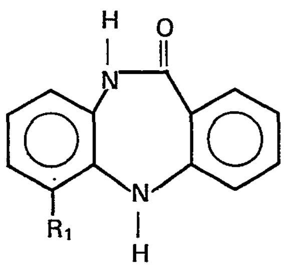

(II)

dans laquelle $R_1$ a la signification indiquée plus haut, au moyen de dérivés d'acides de formule générale III

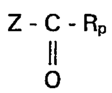

(III)

dans laquelle R est défini comme plus haut et Z représente un groupe nucléofuge, dans un solvant inerte à des températures entre —25 et +130°C ou

b) pour la préparation de composés de formule générale I, dans laquelle R représente un groupe (1-méthyl-1,2,5,6-tétrahydro-4-pyridinyl)-méthyle ou 1-méthyl-1,2,5,6-tétrahydro-4-pyridinyle éventuellement substitué dans le cycle hétérocyclique par un ou deux autres groupes méthyle, on réduit un sel de pyridinium de formule générale Va

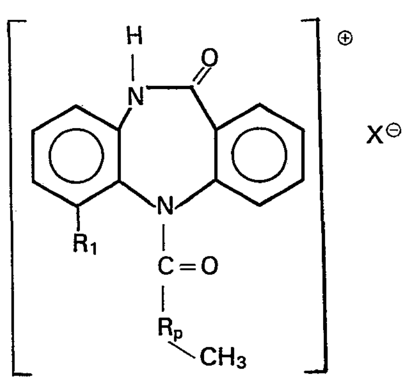

(Va)

dans laquelle $R_1$ est défini comme plus haut, X représente le radical d'acide d'un acide oxygéné fort ou un atome d'halogène et $R_p$ représente un groupe 4-pyridinyle ou (4-pyridinyl)-méthyle, éventuellement substitué par un ou deux autres groupes méthyle, au moyen de borohydrures ou d'alcoxyborohydrures dans des solvants protiques à des températures entre —40 et +50°C, ou

c) pour la préparation de composés de formule générale I, dans laquelle R représente un groupe (1-méthyl-1,2,5,6-tétrahydro-4-pyridinyl)-méthyle, (1-méthyl-1,2,5,6-tétrahydro-4-pipéridinylidène)-méthyle, (2,3-déhydro-8-méthyl-8-aza-bicyclo[3,2,1]oct-3-yl)-méthyle ou (8-méthyl-8-aza-bicyclo[3,2,1]oct-3-ylidène)-méthyle, éventuellement substitué dans le cycle hétérocyclique à six chaînons par un ou deux autres groupes méthyle, on fait réagir une 5-dialcoylphosphonoacétyldibenzodiazépinone de formule générale VII

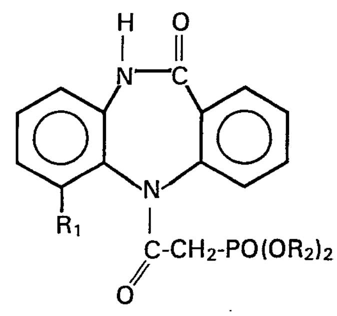

(VII)

dans laquelle $R_1$ est défini comme plus haut et $R_2$ représente un groupe alcoyle avec 1 à 10 atomes de carbone, avec une pipéridinone de formule générale VIII ou une tropinone de formule générale IX

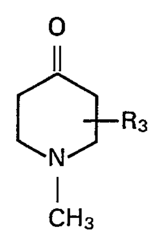

(VIII)

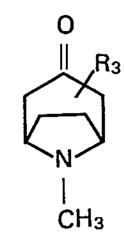

(IX)

dans lesquelles $R_3$ représente un atome d'hydrogène ou le groupe méthyle, en présence d'un hydrure alcalin ou d'un alcoolate alcalin dans un solvant à des températures de 20°C jusqu'à la température d'ébullition du méthyle réactionnel ou

d) pour la préparation de composés de formule générale I dans laquelle R représente un radical (1-méthyl-4-pipéridinyl)-méthyle éventuellement substitué dans le cycle hétérocyclique à six chaînons par un ou deux autres groupes méthyle, ou représente un radical endo- ou exo-(8-méthyl-8-aza-bicyclo[3,2,1]oct-3-yl)-méthyle, on hydrogène catalytiquement une dibenzodiazépinone de formule générale X

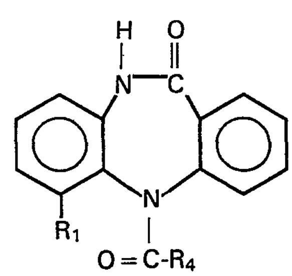

(X)

dans laquelle $R_1$ est défini comme plus haut et $R_4$ représente les groupes suivants

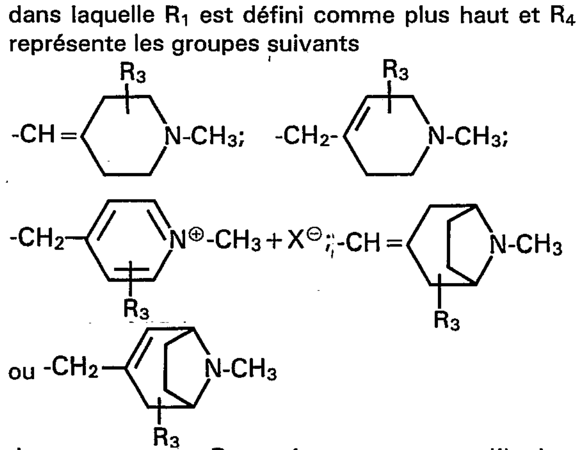

dans ces groupes $R_3$ représente un atome d'hydrogène ou le groupe méthyle et X étant un atome d'halogène, ou

e) on transforme des dibenzodiazépinones de formule générale II

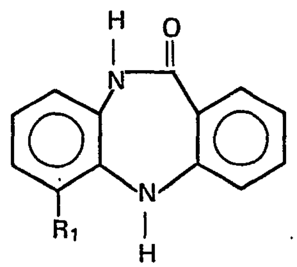

(II)

dans laquelle $R_1$ est défini comme plus haut, dans un solvant organique avec au moins deux équivalents d'un lithiumalcoyle ou lithiumaryle ou lithiumamide, à des températures entre —60 et 0 °C, en leurs sels de dilithium et on fait réagir ensuite ces derniers avec un ester de formule générale XII

$$R-\overset{\overset{O}{\|}}{C}-O-R_5 \quad (XII)$$

dans laquelle R possède les significations mentionnées au début et $R_5$ représente un radical alcoyle avec 1 à 10 atomes de carbone ou un radical aralcoyle avec 7 à 13 atomes de carbone,
et éventuellement in transforme un composé ainsi obtenu de formule générale I ensuite en ses sels avec des acides minéraux ou organiques.

6. Procédé selon la revendication 5a, caractérisé en ce que l'on utilise en tant que dérivé d'acide de formule générale III, un halogénure d'acide, un ester, un anhydride d'acide, un anhydride d'acide mixte ou un sel de N-alcoyl-2-acyloxypyridinium, et en ce que la réaction est effectuée éventuellement dans un solvant inerte en présence d'un agent fixant les acides.

7. Procédé selon la revendication 5b, caractérisé en ce que l'on réduit un sel de pyridinium de formule générale Va au moyen de tétrahydridoborate de sodium ou de potassium ou d'alcoxy-, dialcoxy-, ou trialcoxyborohydrures de sodium ou de potassium à —5 à + 10 °C en présence d'un solvant protique.

8. Procédé pour la revendication des composés de formule générale Va selon la revendication 5b, caractérisé en ce qu'on fait réagir une dibenzodiazépinone de formule générale II

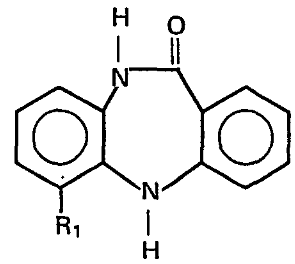

(II)

dans laquelle $R_1$ est défini comme plus haut, avec un agent d'acylation de formule générale IV

$$Z-\underset{\underset{O}{\|}}{C}-R_p \quad (IV)$$

dans laquelle Z représente un groupe nucléofuge et $R_p$ représente un groupe 4-pyridinyle ou (4-pyridinyl)-méthyle. éventuellement substitué par un ou deux groupes méthyle, dans un solvant inerte à des températures allant jusqu'à la température d'ébullition du mélange réactionnel pour obtenir un composé de formule générale V

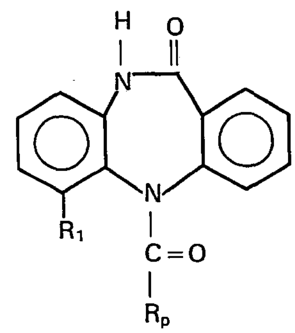

(V)

et en ce qu'ensuite, on méthyle un composé ainsi obtenu au moyen d'un agent de méthylation de formule générale VI

$$H_3C-X \quad (VI)$$

dans laquelle X représente un radical d'acide d'un acide oxygéné fort ou un atome d'halogène, dans un solvant inerte à des températures entre —20 et + 130 °C pour donner le composé correspondant de formule générale Va.

9. Procédé selon la revendication 5d, caractérisé en ce que l'hydrogénation est effectuée au moyen de dioxyde de platine ou de palladium sur charbon ou de nickel de Raney ou de cobalt de Raney, à des températures entre 10 et 40 °C sous une pression d'hydrogène de $10^5$ à $5 . 10^6$ Pa.

10. Utilisation d'un composé de formule générale I selon la revendication 1 pour la préparation de médicaments à activité anti-ulcérogène et inhibiteurs des sécrétions d'acides gastriques.

**Revendications pour l'état contractant:** AT

1. Procédé pour la préparation de dibenzodiazépinones substituées de formule générale I

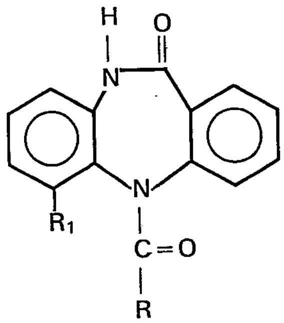

(I)

dans laquelle

$R_1$ représente un atome d'hydrogène ou de chlore et

R représente un groupe (1-méthyl-4-pipéridinyl)-méthyle, (1-méthyl-1,2,5,6-tétrahydro-4-pyridinyl)-méthyle, 1-méthyl-1,2,5,6-tétrahydro-4-pyridinyle, (1-méthyl-4-pipéridinylidène)-méthyle, (2,3-déhydro-8-méthyl-8-aza-bicyclo[3,2,1]oct-3-yl)-méthyle, (8-méthyle-8-aza-bicyclo[3,2,1]oct-3-ylidène)-méthyle éventuellement substitué dans le cycle hétérocyclique par un ou deux autres groupes méthyle, ou représente un groupe endo- ou exo-(8-méthyl-8-azabicyclo[3,2,1]oct-3-yl)-méthyle, et de leurs sels d'addition d'acides avec des acides minéraux ou organiques, caractérisé en ce que:

a) on acyle des dibenzodiazépinones de formule générale II

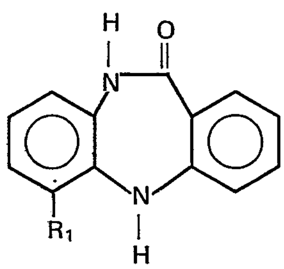

(II)

dans laquelle $R_1$ a la signification indiquée plus haut, au moyen de dérivés d'acides de formule générale III

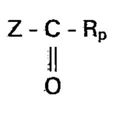

(III)

dans laquelle R est défini comme plus haut et Z représente un groupe nucléofuge, dans un solvant inerte à des températures entre —25 et +130°C ou

b) pour la préparation de composés de formule générale I, dans laquelle R représente un groupe (1-méthyl-1,2,5,6-tétrahydro-4-pyridinyl)-méthyle ou 1-méthyl-1,2,5,6-tétrahydro-4-pyridinyle éventuellement substitué dans le cycle hétérocyclique par un ou deux autres groupes méthyle, on réduit un sel de pyridinium de formule générale Va

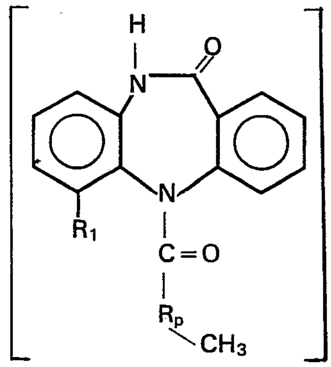

(Va)

dans laquelle $R_1$ est défini comme plus haut, X représente le radical d'acide d'un acide oxygéné fort ou un atome d'halogène et $R_p$ représente un groupe 4-pyridinyle ou (4-pyridinyl)-méthyle, éventuellement substitué par un ou deux autres groupes méthyle, au moyen de borohydrures ou d'alcoxyborohydrures dans des solvants protiques à des températures entre —40 et +50°C, ou

c) pour la préparation de composés de formule générale I, dans laquelle R représente un groupe (1-méthyl-1,2,5,6-tétrahydro-4-pyridinyl)-méthyle, (1-méthyl-1,2,5,6-tétrahydro-4-pipéridinylidène)-méthyle, (2,3-déhydro-8-méthyl-8-aza-bicyclo-[3,2,1]oct-3-yl)-méthyle ou (8-méthyl-8-aza-bicyclo[3,2,1]oct-3-ylidène)-méthyle, éventuellement substitué dans le cycle hétérocyclique à six chaînons par un ou deux autres groupes méthyle, on fait réagir une 5-dialcoylphosphonoacétyldibenzodiazépinone de formule générale VII

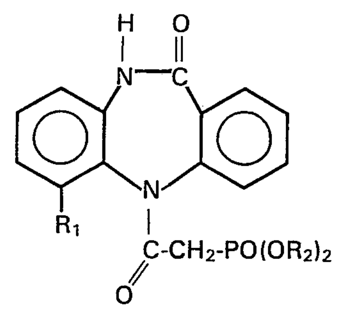

(VII)

dans laquelle $R_1$ est défini comme plus haut et $R_2$ représente un groupe alcoyle avec 1 à 10 atomes de carbone, avec une pipéridinone de formule générale VIII ou une tropinone de formule générale IX

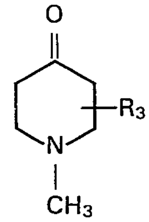

(VIII)

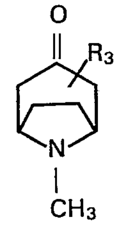

(IX)

dans lesquelles $R_3$ représente un atome d'hydrogène ou le groupe méthyle, en présence d'un hydrure alcalin ou d'un alcoolate alcalin dans un solvant à des températures de 20°C jusqu'à la température d'ébullition du méthyle réactionnel ou

d) pour la préparation de composés de formule générale I dans laquelle R représente un radical

(1-méthyl-4-pipéridinyl)-méthyle éventuellement substitué dans le cycle hétérocyclique à six chaînons par un ou deux autres groupes méthyle, ou représente un radical endo- ou exo-(8-méthyl-8-aza-bicyclo[3,2,1]oct-3-yl)-méthyle, on hydrogène catalytiquement une dibenzodiazépinone de formule générale X

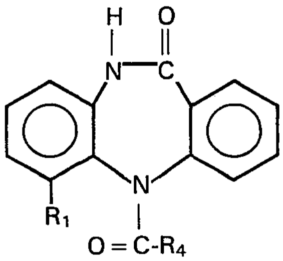

(X)

dans laquelle $R_1$ est défini comme plus haut et $R_4$ représente les groupes suivants

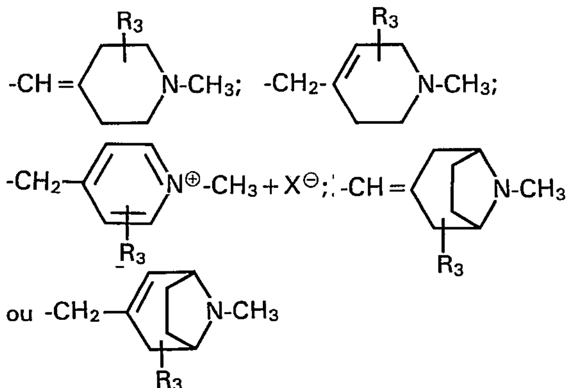

dans ces groupes $R_3$ représentant un atome d'hydrogène ou le groupe méthyle et X étant un atome d'halogène, ou

e) on transforme des dibenzodiazépinones de formule générale II

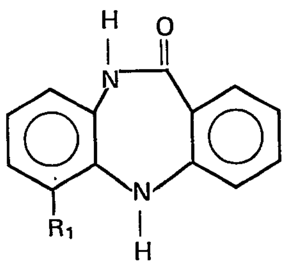

(II)

dans laquelle $R_1$ est défini comme plus haut, dans un solvant organique avec au moins deux équivalents d'un lithiumalcoyle ou lithiumaryle ou lithiumamide, à des températures entre —60 et 0°C, en leurs sels de di-lithium et on fait réagir ensuite ces derniers avec un ester de formule générale XII

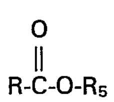

(XII)

dans laquelle R possède les significations mentionnées au début et $R_5$ représente un radical alcoyle avec 1 à 10 atomes de carbone ou un radical aralcoyle avec 7 à 13 atomes de carbone,

et éventuellement on transforme un composé ainsi obtenu de formule générale I ensuite en ses sels avec des acides minéraux ou organiques.

2. Procédé pour la préparation de dibenzodiazépinones substituées de formule générale I

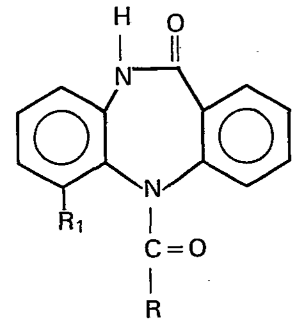

(I)

dans laquelle

$R_1$ représente un atome d'hydrogène ou de chlore et

R représente un groupe (1-méthyl-4-pipéridinyl)--méthyle, (1-méthyl-1,2,5,6-tétrahydro-4-pyridinyl)-méthyle, 1-méthyl-1,2,5,6-tétrahydro-4-pyridinyle, (1-méthyl-4-pipéridinylidène)-méthyle, éventuellement substitué dans le cycle hétérocyclique par un ou deux autres groupes méthyle, ou représente un groupe endo- ou exo-(8-méthyl-8-azabicyclo[3,2,1]oct-3-yl)-méthyle, et de leurs sels d'addition d'acides avec des acides minéraux ou organiques, caractérisé en ce que:

a) on acyle des dibenzodiazépinones de formule générale II

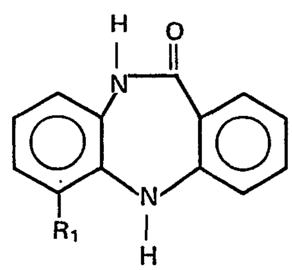

(II)

dans laquelle $R_1$ a la signification indiquée plus haut, au moyen de dérivés d'acides de formule générale III

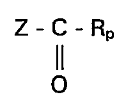

(III)

dans laquelle R est défini comme plus haut et Z représente un groupe nucléofuge, dans un solvant inerte à des températures entre —25 et +130°C ou

b) pour la préparation de composés de formule générale I, dans laquelle R représente un groupe (1-méthyl-1,2,5,6-tétrahydro-4-pyridinyl)-méthyle ou 1-méthyl-1,2,5,6-tétrahydro-4-pyridinyle éventuellement substitué dans le cycle hétérocyclique par un ou deux autres groupes méthyle, on réduite un sel de pyridinium de formule générale Va dans laquelle $R_1$ est défini comme plus haut, X représente le radical d'acide d'un acide oxygéné fort ou un atome d'halogène et $R_p$ représente un groupe

4-pyridinyle ou (4-pyridinyl)-méthyle, éventuellement substitué par un ou deux autres groupes méthyle,

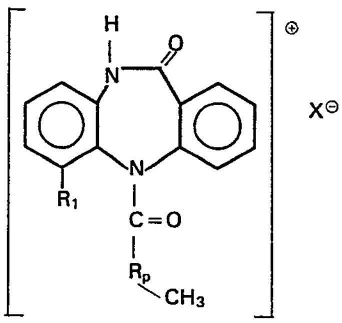

(Va)

au moyen de borohydrures ou d'alcoxyborohydrures dans des solvants protiques à des températures entre —40 et +50°C,

et éventuellement on transforme un composé ainsi obtenu de formule générale I ensuite en ses sels avec des acides minéraux ou organiques.

3. Procédé selon la revendication 1, caractérisé en ce que l'on utilise en tant que dérivé d'acide de formule générale III, un halogénure d'acide, un ester, un anhydride d'acide, un anhydride d'acide mixte ou un sel de N-alcoyl-2-acyloxypyridinium, et en ce que la réaction est effectuée éventuellement dans un solvant inerte en présence d'un agent fixant les acides.

4. Procédé selon la revendication 1b, caractérisé en ce que l'on réduit un sel de pyridinium de formule générale Va au moyen de tétrahydridoborate de sodium ou de potassium ou d'alcoxy-, dialcoxy-, ou trialcoxyborohydrures de sodium ou de potassium à —5 à +10°C en présence d'un solvant protique.

5. Procédé pour la préparation des composés de formule générale Va selon la revendication 1b, caractérisé en ce qu'on fait réagir une dibenzodiazépinone de formule générale II

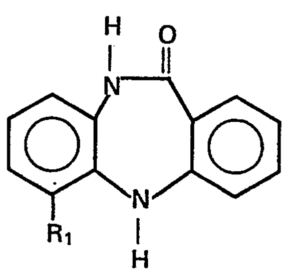

(II)

dans laquelle $R_1$ est défini comme plus haut, avec un agent d'acylation de formule générale IV

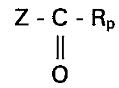

(IV)

dans laquelle Z représente un groupe nucléofuge et $R_p$ représente un groupe 4-pyridinyle ou (4-pyridinyl)-méthyle, éventuellement substitué par un ou deux groupes méthyle, dans un solvant inerte à des températures allant jusqu'à la température d'ébullition du mélange réactionnel pour obtenir un composé de formule générale V

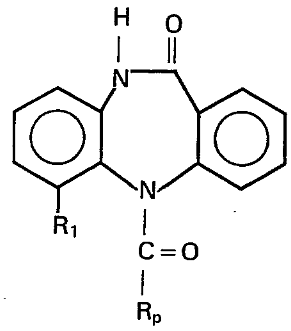

(V)

et en ce qu'ensuite, on méthyle un composé ainsi obtenu au moyen d'un agent de méthylation de formule générale VI

$$H_3C - X \qquad (VI)$$

dans laquelle X représente le radical d'acide d'un acide oxygéné fort ou un atome d'halogène, dans un solvant inerte à des températures entre —20 et +130°C pour donner le composé correspondant de formule générale Va.

6. Procédé selon la revendication 1d, caractérisé en ce que l'hydrogénation est effectuée au moyen de dioxyde de platine ou de palladium sur charbon ou de nickel de Raney ou de cobalt de Raney, à des températures entre 10 et 40°C sous une pression d'hydrogène de $10^5$ à $5.10^6$ Pa.